(19) Europäisches Patentamt
European Patent Office
Office européen des brevets

(11) EP 4 660 190 A1

(12) **EUROPEAN PATENT APPLICATION**
published in accordance with Art. 153(4) EPC

(43) Date of publication:
**10.12.2025 Bulletin 2025/50**

(21) Application number: **24741227.3**

(22) Date of filing: **09.01.2024**

(51) International Patent Classification (IPC):
***C07D 307/83*** *(2006.01)* ***A61K 31/343*** *(2006.01)*
***A61P 9/10*** *(2006.01)* ***A61P 25/00*** *(2006.01)*

(52) Cooperative Patent Classification (CPC):
**A61K 31/343; A61P 9/10; A61P 25/00; C07D 307/83**

(86) International application number:
**PCT/CN2024/071292**

(87) International publication number:
**WO 2024/149228 (18.07.2024 Gazette 2024/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.01.2023 CN 202310037398**
**13.10.2023 CN 202311324351**
**14.11.2023 CN 202311509516**

(71) Applicant: **Jiangsu Chia Tai Fenghai Pharmaceutical Co., Ltd.**
**Yancheng, Jiangsu 224100 (CN)**

(72) Inventors:
- **ZHU, Yongqiang**
  **Yancheng, Jiangsu 224100 (CN)**
- **CHEN, Qi**
  **Yancheng, Jiangsu 224100 (CN)**
- **TANG, Sen**
  **Yancheng, Jiangsu 224100 (CN)**
- **YU, Qince**
  **Yancheng, Jiangsu 224100 (CN)**
- **LIU, Jia**
  **Yancheng, Jiangsu 224100 (CN)**
- **LI, Chenhui**
  **Yancheng, Jiangsu 224100 (CN)**

(74) Representative: **Engelhard, Markus**
**Boehmert & Boehmert Anwaltspartnerschaft mbB**
**Pettenkoferstrasse 22**
**80336 München (DE)**

(54) **PHARMACEUTICALLY ACCEPTABLE SALT OF COMPOUND AND PREPARATION METHOD THEREFOR**

(57) A base addition salt of a compound represented by formula (I) and a salt crystal form thereof, a preparation method therefor and a use thereof, as well as a pharmaceutical composition containing a therapeutically effective amount of the salt or crystal form and a use thereof in the treatment of ischemic brain injury-related diseases, spinal cord injuries, and neurodegenerative diseases.

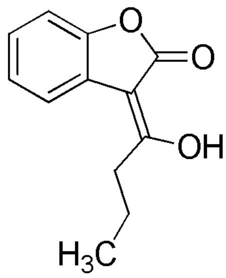

(I)

EP 4 660 190 A1

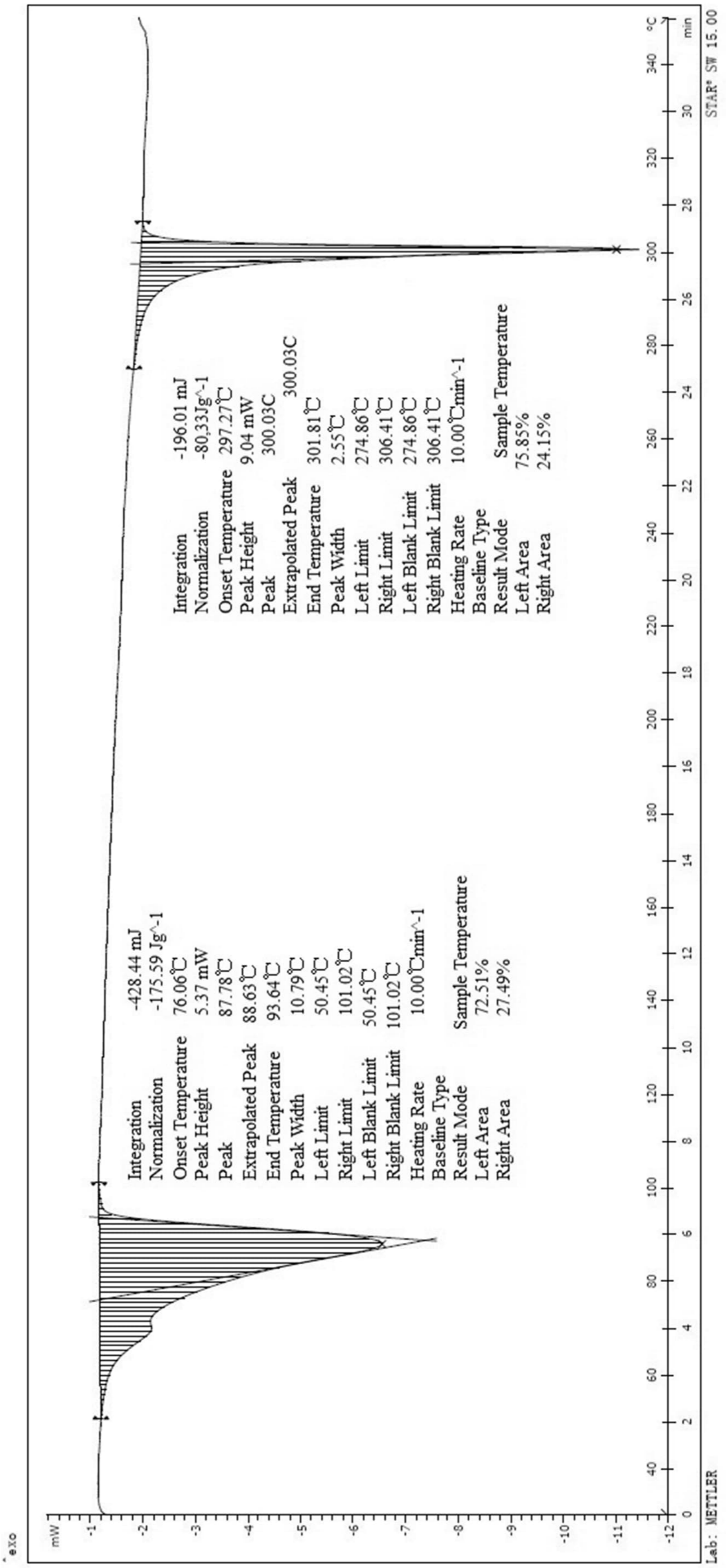

^exo
mW
Integration -428.44 mJ
Normalization -175.59 Jg^-1
Onset Temperature 76.06℃
Peak Height 5.37 mW
Peak 87.78℃
Extrapolated Peak 88.63℃
End Temperature 93.64℃
Peak Width 10.79℃
Left Limit 50.45℃
Right Limit 101.02℃
Left Blank Limit 50.45℃
Right Blank Limit 101.02℃
Heating Rate 10.00℃min^-1
Baseline Type Sample Temperature
Result Mode
Left Area 72.51%
Right Area 27.49%
Integration -196.01 mJ
Normalization -80,33Jg^-1
Onset Temperature 297.27℃
Peak Height 9.04 mW
Peak 300.03C
Extrapolated Peak 300.03C
End Temperature 301.81℃
Peak Width 2.55℃
Left Limit 274.86℃
Right Limit 306.41℃
Left Blank Limit 274.86℃
Right Blank Limit 306.41℃
Heating Rate 10.00℃min^-1
Baseline Type Sample Temperature
Result Mode
Left Area 75.85%
Right Area 24.15%
°C
min
Lab: METTLER
STAR^e SW 15.00


Fig. 1

## Description

### TECHNICAL FIELD

The present invention belongs to the field of biomedicine, and specifically relates to pharmaceutically acceptable salts, crystal forms, and pharmaceutical compositions of a compound having anti-cerebral ischemia, anti-post-ischemic inflammation, and anti-convulsive activities, and capable of improving neurological functional impairment in patients with ischemic stroke, spinal cord injury, or neurodegenerative diseases, and further relates to the use and preparation methods of the aforementioned salts, salt crystal forms, and pharmaceutical compositions for treating ischemic brain injury-related diseases.

### BACKGROUND ART

White matter an essential component of the central nervous system, consists of densely aggregated nerve fibers. White matter lesions (WML) are usually caused by reduced blood flow and insufficient oxygen supply to the blood vessels. In the presence of dysfunction (insufficiency) in the blood supply to the brain, a series of symptoms ensue due to the difficulty in meeting the metabolic needs of brain tissues. The clinical manifestations include dizziness, headache, numbness of limbs, or transient loss of consciousness, and in severe cases irreversible brain damage or death may occur. Diseases related to cerebral ischemia include transient ischemic attack (TIA), ischemic cerebral stroke (cerebral infarction), moyamoya disease, chronic cerebral circulatory insufficiency, and the like. These conditions are also contributing factors to the decline in cognitive function and vascular dementia in patients.

Spinal cord injury (SCI), one of the leading causes of disability in modern society, is often accompanied by symptoms such as limb sensory impairment and motor dysfunction. As an injury to the central nervous system, SCI affects non-regenerative tissues, and the resulting functional impairments are often irreversible and difficult to treat. Traumatic brain injury is a major cause of cognitive impairment in patients with SCI. SCI imposes severe physical and psychological burdens on patients and results in substantial societal and economic costs.

Parkinson's Disease (PD) is a common neurodegenerative disease and has become a major global health concern. Its typical clinical features include motor symptoms such as bradykinesia, resting tremor, and postural instability. The fundamental cause is the loss, injury and death of dopaminergic neurons in the midbrain. The current standard therapy is dopamine replacement therapy, which addresses the symptoms rather than the root cause and comes with significant side effects.

Although several drugs are currently used to treat central nervous system (CNS) injury-related conditions, few demonstrate robust therapeutic efficacy. Nimodipine, a calcium channel blocker currently in clinical use, has shown some preventive benefit in cerebral ischemia, but its therapeutic efficacy remains inconclusive. Neuronal damage and necrosis in the brain can result in widespread neurological dysfunction and significantly impair patients' quality of life. Therefore, there remains an unmet need for compounds with superior efficacy that exhibit anti-ischemic, anti-inflammatory (post-ischemia), and anticonvulsant properties, and that are capable of improving neurological function, enhancing memory, and protecting neurons as well as maintaining the integrity of the blood-brain barrier.

The present invention relates to a compound of formula (I), (Z)-3-(1-hydroxybutenyl)benzofuran-2-one for the treatment of ischemic brain injury, spinal cord injury, and neurodegenerative diseases. In subsequent research and development, comprehensive studies were carried out on its pharmaceutically acceptable salts to improve processing characteristics such as filtration and drying, and to ensure suitability for storage and long-term stability. The aim is to identify the optimal salt form to ensure the quality, safety, efficacy, and application of the drug.

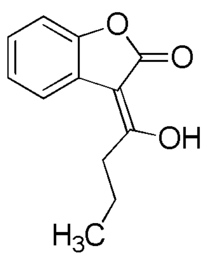

(I)

### SUMMARY

The present invention provides pharmaceutically acceptable salts and crystal forms of a compound having anti-cerebral ischemia, anti-post-ischemic inflammation, and anti-convulsive activities, which is capable of ameliorating neurological functional impairment in patients with ischemic stroke or spinal cord injury, as well as cognitive dysfunction in patients with neurodegenerative diseases, and also relates to methods for the preparation thereof.

The present invention provides pharmaceutically acceptable salts of a compound of formula (I).

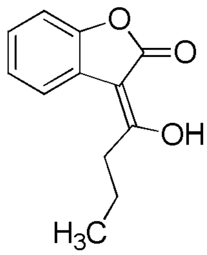

(I) ,

wherein the pharmaceutically acceptable salts are selected from sodium salts, potassium salts, magnesium salts, calcium salts, piperazine salts, ethanolamine salts, meglumine salts, and tromethamine salts. These pharmaceutically acceptable salts may also be selected from the hydrates of the salts, including monohydrates, dihydrates, trihydrates, or hemihydrates. The salt is selected from sodium salts, potassium salts, magnesium salts, calcium salts, piperazine salts, ethanolamine salts, meglumine salts, and tromethamine salts.

The present invention further provides the pharmaceutically acceptable salt of a compound of formula (I), wherein the pharmaceutically acceptable salt is (Z)-3-(1-hydroxybutenyl)benzofuran-2-one sodium salt.

The present invention further provides the pharmaceutically acceptable salt of a compound of formula (I), wherein the pharmaceutically acceptable salt is (Z)-3-(1-hydroxybutenyl)benzofuran-2-one sodium monohydrate.

The present invention further provides the pharmaceutically acceptable salt of a compound of formula (I), wherein the pharmaceutically acceptable salt is(Z)-3-(1-hydroxybutenyl)benzofuran-2-one potassium salt.

The present invention further provides the pharmaceutically acceptable salt of a compound of formula (I), wherein the pharmaceutically acceptable salt is (Z)-3-(1-hydroxybutylidene)benzofuran-2-one magnesium salt.

The present invention further provides the pharmaceutically acceptable salt of a compound of formula (I), wherein the pharmaceutically acceptable salt is (Z)-3-(1-hydroxybutenyl)benzofuran-2-one calcium salt.

The present invention further provides the pharmaceutically acceptable salt of a compound of formula (I), wherein the pharmaceutically acceptable salt is (Z)-3-(1-hydroxybutylidene)benzofuran-2-one piperazine salt.

The present invention further provides the pharmaceutically acceptable salt of a compound of formula (I), wherein the pharmaceutically acceptable salt is (Z)-3-(1-hydroxybutenyl)benzofuran-2-one ethanolamine salt.

The present invention further provides the pharmaceutically acceptable salt of a compound of formula (I), wherein the pharmaceutically acceptable salt is (Z)-3-(1-hydroxybutenyl)benzofuran-2-one meglumine salt.

The present invention further provides the pharmaceutically acceptable salt of a compound of formula (I), wherein the pharmaceutically acceptable salt is (Z)-3-(1-hydroxybutenyl)benzofuran-2-one tromethamine salt.

The present invention also provides a method for preparing the pharmaceutically acceptable salt, comprising a step of forming a salt from a compound of formula (I) with a base.

The solvent used for the salt formation reaction is selected from at least one of the following: methanol, ethanol, methyl acetate, ethyl acetate, isopropyl acetate, n-butyl acetate, acetonitrile, isopropanol, acetone, tetrahydrofuran, methyl isobutyl ketone, n-heptane, dichloromethane, toluene, isopropyl ether, methyl tert-butyl ether, n-butanol, or water.

The present invention also provides a pharmaceutical composition, which comprises the compound of formula (I) or the pharmaceutically acceptable salt thereof described herein and one or more pharmaceutically acceptable carriers, diluents, or excipients.

The present invention also provides use of the compound of formula (I) or the pharmaceutically acceptable salt thereof described herein in preparing a medicament.

The present invention also provides use of the composition described herein in preparing a medicament for treating and/or preventing diseases related to cerebral ischemic injury.

The ischemic brain injury-related diseases described herein include but are not limited to cerebral ischemia, ischemic stroke, vascular dementia, post-ischemic inflammation, convulsion, ischemic neuronal injury or necrosis.

The present invention also provides use of the composition described herein in preparing a medicament for treating and/or preventing neuromuscular diseases.

The neuromuscular diseases described herein refer to amyotrophic lateral sclerosis (ALS).

The present invention also provides use of the composition described herein in preparing a medicament for treating and/or preventing neurological functional impairment-related diseases.

The neurological functional impairment-related disease described herein include but not limited to the spinal cord injury and necrosis.

The present invention also provides use of the composition described herein in preparing a medicament for treating and/or preventing neurodegenerative diseases.

The neurodegenerative disease described herein include but not limited to the Parkinson's disease.

The pharmaceutically acceptable salts of the compound described herein are easy to handle, filter, and dry, and they are stable for long-term storage. These properties make them suitable for industrial development, ensuring the quality, safety, and efficacy of the drug.

## BRIEF DESCRIPTION OF THE DRAWINGS

FIG. 1 shows the Differential Scanning Calorimetry (DSC) thermogram of the sodium salt monohydrate.

FIG. 2 shows the Thermogravimetric Analysis (TGA) of the sodium salt monohydrate.

FIG. 3 shows the effect of the tromethamine salt from Example 10 on the survival of SOD1 G93A ALS mice.

FIG. 4 shows the results of the pole test for MPTP-induced Parkinson's disease model mice treated with the tromethamine salt from Example 10 (Note: The data in the figure are expressed as Mean $\pm$ SEM). $^{\&}P<0.05$ indicates a statistically significant difference compared to the normal control group, and $^{\#}P<0.05$ indicates a statistically significant difference compared to the model control group.

FIG. 5 shows the results of the grip strength test for MPTP-induced Parkinson's disease model mice treated with the tromethamine salt from Example 10 (Note: Data are expressed as Mean $\pm$ SEM). $^{\&}P<0.05$ indicates a statistically significant difference compared to the normal control group;

FIG. 6 shows the results of DOPA level detection results in the striatum (Note: The data in the figure are expressed as Mean $\pm$ SEM). $^{\&}P<0.05$ indicates a statistically significant difference compared to the normal control group, and $^{\#}P<0.05$ indicates a statistically significant difference compared to the model control group.

FIG. 7 shows the statistical results of TH-positive fiber content in the striatum (Panel A: 2.5$\times$ microscopic image of TH immunohistochemical staining in mouse striatum, with brownish-yellow positive signals. Panel B: Quantification of TH-positive areas in mouse striatum. Three sections per animal were analyzed. The data in the figure are expressed as Mean $\pm$ SEM). $^{\&}P<0.05$ indicates a statistically significant difference compared to the normal control group, and $^{\#}P<0.05$ indicates a statistically significant difference compared to the model control group.

FIG. 8 shows the statistical results of TH-positive cell counts in the substantia nigra pars compacta Panel A:10$\times$ microscopic view of TH immunofluorescence staining in the substantia nigra pars compacta. Panel B: Quantification of TH-positive cells. Three sections per animal were analyzed. The data in the figure are expressed as Mean $\pm$ SEM). The data in the figure are expressed as Mean $\pm$ SEM. $^{\&}P<0.05$ indicates a statistically significant difference compared to the normal control group, and $^{\#}P<0.05$ indicates a statistically significant difference compared to the model control group.

FIG. 9 shows representative images of the pathological changes observed in Example 10 treated with the tromethamine salt (50$\times$ and 200$\times$ magnification).

FIG. 10 shows representative images showing the effect of the tromethamine salt from Example 10 on NF200 expression (400$\times$ magnification).

FIG. 11 shows the Gene Set Enrichment Analysis (GSEA) results showing the effects of the tromethamine salt from Example 10 on the transcriptomic profile of injured spinal cord tissue.

## DETAILED DESCRIPTION

The present invention is further illustrated below with reference to specific examples, in accordance with general technical knowledge and conventional practice in the art. The following examples are merely exemplary of the present invention and should not be construed as limiting the present invention, and it is apparent to those skilled in the art that several modifications can be made without departing from the scope of the present invention and these modifications shall also fall within the protection scope of the present invention.

Example 1: preparation of (Z)-3-(1-hydroxybutenyl)benzofuran-2-one

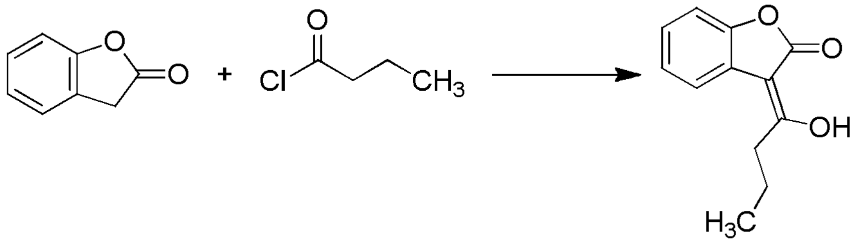
(I)

The organic phase was washed with 0.1 N hydrochloric acid to be acidic (pH < 2), dried over anhydrous sodium sulfate (5 g) for 0.5 h, filtered, and concentrated to give an oil product, which was then purified by high pressure preparative chromatography, and the purified solution was freeze-dried to afford the compound of formula (I) (Z)-3-(1-hydroxybutenyl) benzofuran-2-one (2.5 g, 32.9%) as a white solid.

$^1$HNMR (CDCl3, 400 MHz): $\delta$12.02 (s, 1H), 7.35-7.33 (d, 1H), 7.28-7.17 (m, 3H), 2.76-2.73 (m, 2H), 1.90-1.80 (m, 2H), 1.12-1.08 (m, 3H).

$^1$HNMR ($CDCl_3$+$D_2O$, 400MHz): $\delta$7.34-7.32 (d, 1H), 7.28-7.16 (m, 3H), 2.76-2.72 (m, 2H), 1.89-1.80 (m, 2H), 1.12-1.08 (m, 3H)

Example 2: preparation of sodium salt

(Z)-3-(1-hydroxybutenyl)benzofuran-2-one (10 g, 0.05 mol, 1.0 eq.) was dissolved in methanol (100 mL). After dissolution, sodium hydroxide (1.95 g, 0.05 mol, 1.0 eq.) was added, and the mixture was stirred for 1 hour. The reaction mixture was then concentrated, and acetone (200 mL) was added. The mixture was heated to 50°C to dissolve, then cooled to room temperature and maintained for 2 hours. The resulting solid was filtered and dried to yield 11 g of off-white solid.

Example 3: preparation of sodium salt monohydrate

(Z)-3-(1-hydroxybutenyl)benzofuran-2-one sodium salt, the off-white solid product from Example 1 (5 g, 0.22 mol, 1.0 eq.), was dissolved in water (20 mL). The mixture was heated to 60°C to dissolve the solid, then cooled to 20°C and maintained for 2 hours. The resulting solid was filtered and dried to obtain 3 g of a white solid. The Differential Scanning Calorimetry (DSC) thermogram of this powdered solid is shown in Figure 1, and its corresponding Thermogravimetric Analysis (TGA) curve is shown in Figure 2.

Example 4: preparation of potassium salt

(Z)-3-(1-hydroxybutenyl)benzofuran-2-one (1 g, 5 mmol, 1.0 eq.) was dissolved in acetone (10 mL). After dissolution, potassium hydroxide (0.27 g, 5 mmol, 1.0 eq.) was added, and the mixture was stirred for 0.5 hours. The solid was filtered and dried to obtain 0.9 g of a white solid.

Example 5: preparation of magnesium salt

(Z)-3-(1-hydroxybutenyl)benzofuran-2-one (1 g, 5 mmol, 1.0 eq.) was dissolved in methyl acetate (10 mL). After dissolution, magnesium chloride (0.46 g, 5 mmol, 1.0 eq.) was added, and the mixture was stirred for 1.5 hours. The solid was filtered and dried to obtain 0.75 g of a white solid.

Example 6: preparation of calcium salt

(Z)-3-(1-hydroxybutenyl)benzofuran-2-one (1 g, 5 mmol, 1.0 eq.) was dissolved in acetone (10 mL). After dissolution, calcium chloride (0.54 g, 5 mmol, 1.0 eq.) was added, and the mixture was stirred for 1 hour. The solid was filtered and dried to obtain 1.05 g of a white solid.

Example 7: preparation of piperazine salt

(Z)-3-(1-hydroxybutenyl)benzofuran-2-one (2 g, 10 mmol, 1.0 eq.) was dissolved in methyl isobutyl ketone (20 mL). After dissolution, piperazine (0.84 g, 10 mmol, 1.0 eq.) was added, and the mixture was stirred for 1 hour. The solid

was filtered and dried to obtain 2.20 g of a white solid.

Example 8: preparation of ethanolamine salt

(Z)-3-(1-Hydroxybutenyl)benzofuran-2-one (2 g, 10 mmol, 1.0 eq.) was dissolved in ethanol (20 mL). After dissolution, ethanolamine (0.60 g, 10 mmol, 1.0 eq.) was added, and the mixture was stirred for 3 hours. The solid was filtered and dried to obtain 1.88 g of a white solid.

Example 9: preparation of meglumine salt

(Z)-3-(1-Hydroxybutenyl)benzofuran-2-one (2 g, 10 mmol, 1.0 eq.) was dissolved in acetonitrile (20 mL). After dissolution, meglumine (1.90 g, 10 mmol, 1.0 eq.) was added, and the mixture was stirred for 2 hours. The solid was filtered and dried to obtain 1.94 g of a white solid.

Example 10: preparation of tromethamine salt

(Z)-3-(1-Hydroxybutenyl)benzofuran-2-one (20 g, 0.1 mol, 1.0 eq.) was dissolved in isopropanol (200 mL). After dissolution, tromethamine (11.8 g, 0.1 mol, 1.0 eq.) was added, and the mixture was stirred for 2 hours. The solid was filtered and dried to obtain 23 g of a white solid.

Experimental results have shown that the salts described in Examples 2-10 can all release the compound (Z)-3-(1-hydroxybutenyl)benzofuran-2-one under conventional experimental conditions.

Example 11: study on the Solubility and Hygroscopicity of the Crystal Form of the Tromethamine Salt

The tromethamine salt sample obtained in Example 10 was evaluated for its solubility at 20 °C, 25 °C, and 30 °C. The test results are shown in Table 1.

Table 1 Solubility data of the tromethamine salt in water at different temperatures

| Sample weight (from Example 10) | Temperature | Volume of water for dissolution |
|---|---|---|
| 1 g | 20°C | 25 ml |
| | 25°C | 20 ml |
| | 30°C | 14 ml |

According to the Guiding Principles for Drug Hygroscopicity Testing set forth in Section 9103 of the Chinese Pharmacopoeia:

1. One day before the test, take a dry, stoppered glass weighing bottle (outer diameter 50 mm, height 15 mm), and place it in a suitable constant temperature desiccator maintained at 25°C ± 1°C (with saturated ammonium chloride or ammonium sulfate solution placed at the bottom) or in a climate chamber set to 25 °C ± 1 °C and 80% ± 2% relative humidity. Accurately weigh and record the weight ($m_1$).
2. Take an appropriate amount of the test sample and spread it evenly in the above-mentioned weighing bottle, with a thickness of approximately 1 mm. Accurately weigh and record the weight ($m_2$).
3. Leave the weighing bottle open and place it together with its cap under the above-mentioned constant temperature and humidity conditions for 24 hours.
4. Cover the weighing bottle, and accurately weigh and record the weight ($m_3$).
5. Description of hygroscopicity characteristics and definition of hygroscopic weight gain

Deliquescence: absorbs sufficient moisture to form a liquid.
Highly hygroscopic: hygroscopic weight gain of no less than 15%.
Hygroscopic: Weight gain due to moisture absorption is less than 15% but not less than 2%.
Slightly hygroscopic: Weight gain due to moisture absorption is less than 2% but not less than 0.2%.
Non-hygroscopic or almost non-hygroscopic: Weight gain due to moisture absorption is less than 0.2%.
Test results: $m_1$=28.7554; $m_2$=29.7773; $m_3$=29.7773.

Calculation formula: Weight gain percentage = $\frac{m3-m2}{m2-m1} \times 100\%$

The appearance of the tromethamine salt sample from Example 10 remained unchanged, with a weight gain percentage of 0.0%, indicating that the sample was non-hygroscopic.

Example 12: Stability Study

The stability of the sodium salt sample obtained in Example 2 was evaluated under high temperature, high humidity, and light exposure conditions. The sampling observation period was 10 days. The results are summarized in Table 2.

Table 2: Stability Study of Sodium Salt (Example 2 Sample) under Influence Factors

| Sample | Influence factors (10d) | Impurities | | |
|---|---|---|---|---|
| | | RRT1.41 | RRT1.47 | Total impurities |
| Example 2 | 0 h | 0.10 | 0.11 | 0.21 |
| | High temperature 60°C | 0.14 | 0.09 | 0.23 |
| | High Humidity 75% | 0.12 | 0.10 | 0.22 |
| | Light | 0.07 | 0.09 | 0.16 |

The stability of the potassium, magnesium, calcium, piperazine, ethanolamine, and meglumine salt samples obtained in Examples 4 to 9 was evaluated under high temperature, high humidity, and light conditions. The sampling observation period was 10 days. The results are summarized in Table 3.

Table 3: Stability Study of Potassium Salt, Magnesium Salt, Calcium Salt, Piperazine Salt, Ethanolamine Salt, and Meglumine Salt (Examples 4 to 9 Samples) under Stress Conditions

| Samples | Influence factors (10 d) | Appearance | Purity |
|---|---|---|---|
| Example 4 (Potassium Salt) | 0 h | White solid | 99.41% |
| | High temperature 40°C | White solid | 99.66% |
| | High temperature 60°C | White solid | 99.58% |
| | High Humidity 75% | White solid | 99.34% |
| | High Humidity 92.5% | White solid | 99.47% |
| | Light | White solid | 99.41 % |
| Example 5 (Magnesium Salt) | 0 h | White solid | 99.67% |
| | High temperature 40°C | White solid | 99.82% |
| | High temperature 60°C | White solid | 99.58% |
| | High Humidity 75% | White solid | 99.83% |
| | High Humidity 92.5% | White solid | 99.79% |
| | Light | White solid | 99.72% |
| Example 6 (Calcium Salt) | 0 h | White solid | 99.42% |
| | High temperature 40°C | White solid | 99.58% |
| | High temperature 60°C | White solid | 99.68% |
| | High Humidity 75% | White solid | 99.56% |
| | High Humidity 92.5% | White solid | 99.44% |
| | Light | White solid | 99.43% |

(continued)

| Samples | Influence factors (10 d) | Appearance | Purity |
|---|---|---|---|
| Example 7 (Piperazine Salt) | 0 h | Light yellow solid | 99.59% |
| | High temperature 40°C | Light yellow solid | 99.67% |
| | High temperature 60°C | Light yellow solid | 99.72% |
| | High Humidity 75% | Light yellow solid | 99.66% |
| | High Humidity 92.5% | Light yellow solid | 99.54% |
| | Light | Light yellow solid | 99.43% |
| Example 8 (Ethanolamine Salt) | 0 h | White solid | 99.19% |
| | High temperature 40°C | White solid | 99.75% |
| | High temperature 60°C | White solid | 99.75% |
| | High Humidity 75% | White solid | 99.71% |
| | High Humidity 92.5% | White solid | 99.79% |
| | Light | White solid | 99.78% |
| Example 9 (Meglumine Salt) | 0 h | White solid | 99.53% |
| | High temperature 40°C | White solid | 99.45% |
| | High temperature 60°C | White solid | 98.04% |
| | High Humidity 75% | White solid | 99.01 % |
| | High Humidity 92.5% | Yellow oily liquid | 98.62% |
| | Light | White solid | 99.60% |

The stability of the tromethamine salt sample obtained in Example 10 was evaluated under high temperature (60 °C), light exposure (unwrapped and packaged), and high humidity (92.5% and 75%) conditions.

Table 4: Stability Study of Tromethamine Salt (Example 10 Sample) under Influence Factors

| Samples | Influence Factors Conditions (10 d) | Total impurities |
|---|---|---|
| Example 10 | 0 h | 0.08 |
| | High temperature 60°C | 0.03 |
| | Light (unwrapped) | 0.03 |
| | Light (packaged) | 0.04 |
| | High Humidity 92.5% | 0.04 |
| | High Humidity 75% | 0.04 |

Conclusion: The stress condition study demonstrated that the tromethamine salt exhibited good chemical stability under high temperature (60°C), light exposure (unwrapped), light exposure (packaged), and high humidity (92.5%, 75%) conditions.

**Biological Assays:**

**Experiment 1: Compound Activity Assay**

The following studies demonstrate that the compound of formula (I), as described in Example 1, can alleviate neurological deficits associated with cerebral ischemia. The results further indicate that the compound of formula (I) exhibits favorable oral bioavailability and pharmacological activity.

1. Pharmacokinetic test in rats

Male SD rats (weighing 180-260g) were administered with the compound of formula (I) intravenously at a dose of 1.0 mg/kg via tail vein injection, and orally at a dose of 10.0 mg/kg. Each group consisted of 3 animals. The solvent for administration was a solution of 5% DMSO + 5% polyethoxylated castor oil (Cremophor EL) in normal saline. The rats were fasted for about 12 h before administration and were given ad libitum access to food 4 h after administration, and water was available all the time. Blood was collected from the orbit at about 0.2 mL before administration and 5 min, 15 min, 30 min, 1 h, 2 h, 4 h, 6 h, 8 h, and 24 h after administration. The samples were placed in EDTA-K2 anticoagulant EP tubes in an ice bath, and subjected to low-speed centrifugation at 4°C and 3500 rpm for 10 min to isolate plasma, which was then stored at -20°C before analysis. The plasma concentration of the compound of formula (I) was determined using liquid chromatography-tandem mass spectrometry (LC-MS/MS). Pharmacokinetic parameters were calculated from the analysis results of the samples using WinNonlin software.

As shown in Table 5, following oral administration, the pharmacokinetic parameters of the compound of formula (I) demonstrated prolonged systemic retention and higher bioavailability compared to reported values for butylphthalide (NBP) in the literature (Wang Ningning, Li Yue, Li Xiaohong, Jiang Mingyan, RP-HPLC determination of the content of 3-n-butylphthalide in plasma of rats and pharmacokinetics thereof, Chinese Journal of New Drugs and Clinical Remedies, Dec. 2012, Vol. 31, Issue 12, pp.743-747). The higher-than-expected oral bioavailability (>100%) observed for compound (I) may be attributed to nonlinear pharmacokinetics.

Table 5 Pharmacokinetic parameters

| Parameter | Unit | Compound (I) | | Butylphthalide (value in literature[1]) | |
|---|---|---|---|---|---|
| | | i.v. 1 mg/kg | p.o. 10 mg/kg | i.v. 10 mg/kg | p.o. 60 mg/kg |
| $T_{1/2}$ | h | 7.5 | 9.6 | 2.2 | 2.83 |
| $T_{max}$ | h | / | 0.5 | / | 0.7 |
| $C_{max}$ | ng/mL | / | 56533.3 | / | 2900.0 |
| $MRT_{inf}$ | h | 9.8 | 13.2 | 2.4 | 3.8 |
| $AUC_{0-t}$ | h*ng/mL | 52515.0 | 702610.0 | 7230.0 | 7110.0 |
| $AUC_{0-inf}$ | h*ng/mL | 59248.7 | 854122.3 | 8620.0 | 8560.0 |
| F | % | 144.2 | | 16.6* | |
| Calculated based on the literature value of AUC0-inf listed in the table | | | | | |

2: Distribution in brain tissues of rats

Male SD rats (weighing 200-270g) were orally given compound (I) and butylphthalide (NBP) at a dose of 20mg/kg separately. Plasma and brain tissue samples were collected from the animals at 0.5h, 1h, 4h, and 24h after administration. Collection of plasma: 0.2mL of whole blood was collected by using an EP tube containing EDTA-K2 and centrifuged at 3500 g for 10min, and then the upper plasma was collected and stored at -20°C. Collection of brain tissue: after the animals were euthanized, a proper amount of brain tissues were weighed out and homogenized with methanol in water (80%, w/v) at a ratio of 1:4. The concentration of compound in the plasma and brain tissue samples was quantified by liquid chromatography-tandem mass spectrometry (LC-MS/MS).

As shown in Table 6, the compound of formula (I) exhibited higher concentrations in both plasma and brain tissues of rats following oral administration.

Table 6. Concentration of compound in rat plasma and brain tissues

| Samplin g time | Compound (I) 20mg/kg p.o. | | NBP 20mg/kg p.o. | |
|---|---|---|---|---|
| | Measured value in plasma Concentration (ng/mL) | Measured value in brain tissues Concentration (ng/g) | Measured value in plasma Concentration (ng/mL) | Measured value in brain tissues Concentration (ng/g) |
| 0.5 h | 241333.3 | 8733.3 | 26.0 | 28.5 |
| 1 h | 192333.3 | 5726.7 | 19.1 | 22.8 |

(continued)

| Sampling time | Compound (I) 20mg/kg p.o. | | NBP 20mg/kg p.o. | |
|---|---|---|---|---|
| | Measured value in plasma Concentration (ng/mL) | Measured value in brain tissues Concentration (ng/g) | Measured value in plasma Concentration (ng/mL) | Measured value in brain tissues Concentration (ng/g) |
| 4h | 182333.3 | 5030.0 | 7.2 | 10.2 |
| 24 h | 23500.0 | 285.3 | 1.7 | NA |
| NA: below the lower limit of quantification | | | | |

3: Pharmacodynamic study in Rat Middle Cerebral Artery Occlusion (MCAO) Model (Single-Dose Administration) Middle cerebral artery occlusion (MCAO) models of SD rats were constructed using the suture occlusion method to evaluate the protection effect of the compound on neurons of rats after cerebral ischemia-reperfusion. After anaesthesia induction with 3.0% isoflurane in SD rats (240-270g), the operation was performed, and the right-side neck area was dissected to reveal the right common carotid artery (CCA), external carotid artery (ECA) and internal carotid artery (ICA). The ECA was ligated. The ICA was temporarily occluded. Sutures were threaded at both the proximal end and the distal end of CCA. The distal end was securely ligated, and a loosely tied knot was placed at the proximal end. A small incision was made between the two ligatures on the CCA. A No. 4-0 suture was inserted through the incision of CCA and slowly and gently pushed into the ICA. The threading was halted temporarily when reaching the artery clamp of ICA. The pre-ligation suture was further tightened. The artery clamp blocking the blood flow in the ICA was removed, allowing the suture occlusion to be advanced into the ICA until it entered the intracranial region. When the suture was inserted about 18mm away from the bifurcation of the CCA, slight resistance was felt, indicating that the tip of the suture had reached the anterior cerebral artery (ACA), and the opening of the middle cerebral artery (MCA) was occluded. The insertion was stopped at the moment, and the time was recorded. The artery clamp on the CCA was removed and the incision was closed after no active bleeding was observed. The ischemic rats were placed at room temperature to keep their body temperature at 37°C, and anesthesia induction could be carried out 120min later. The suture was slowly and gradually withdrawn when the rats were maintained under anesthesia, so that the proximal end of the suture returned to the ECA, thereby initiating reperfusion of the middle cerebral artery. Animals were administered for a single dose immediately after reperfusion (within 10min). 3 groups were established, namely a model control group, a Compound (I) i.v. group (30mg/kg), and a Compound (I) p.o. group (60mg/kg). Animals were euthanized 24 h after ischemia reperfusion, and the brains were rapidly taken, frozen, and dissected for TTC staining. The normal tissues were rose-red after staining, and the infarcted tissues were white. The infarct size (%) percentage, calculated as the weight of infarcted tissues to the weight of the whole brain, is used to evaluate the degree of cerebral ischemic injury in rats.

On the first day post-surgery, TTC staining analysis revealed that the cerebral infarct size in the model control group is 21.63±5.66%, and the cerebral infarct sizes of the compound (I) i.v. group and the compound (I) p.o. group are 13.61±3.66% and 14.88±5.11%, respectively. It shows that both the compound (I) i.v. group and the compound (I) p.o. group can significantly reduce the cerebral infarct size of animals ($p = 0.0025$ and $p = 0.0389$); meanwhile, the cerebral infarction inhibition rates of the animals in the compound (I) i.v. group and the compound (I) p.o. group are 37.1% and 31.2%, respectively. These results collectively demonstrate that the compound (I) can effectively ameliorate the outcome of cerebral infarction in rats, as detailed in Table 7.

Table 7 Cerebral infarct size and cerebral infarction inhibition rate of experimental animals

| Group | Infarct Size (%) | Cerebral infarction inhibition rate (%) |
|---|---|---|
| Model control group (N = 13) | 21.63± 5.66 | - |
| Compound (I) i.v. group (N = 12) | 13.61±3.66** | 37.1 |
| Compound (I) p.o. group (N = 10) | 14.88±5.11* | 31.2 |

Note: the data in the table are all expressed as Mean±standard deviation (Mean±SD); "N" represents the number of animals in each group for statistical analysis; "-" represents no data for this item; * represents $p < 0.05$ compared with animals in the model control group; ** represents $p < 0.01$ compared with animals in the model control group.

4. Middle and long-term pharmacodynamic study of compound herein in rat cerebral stroke model

Middle cerebral artery occlusion (MCAO) models of SD rata were constructed by using the suture occlusion

method. The rats were administered with the test compound for 28 consecutive days. The pharmacodynamic evaluation on the test compound for cerebral stroke was obtained based on general observation and neurobehavioral assessment.

After anesthesia induction with 3.0% isoflurane in SD rats (240-280g), the operation was performed and the right common carotid artery (CCA), and the right-side neck area was dissected to reveal the right common carotid artery (CCA), external carotid artery (ECA) and internal carotid artery (ICA). The ECA was ligated. The ICA was temporarily occluded. Sutures were threaded at both the proximal end and the distal end of CCA. The distal end was securely ligated, and a loosely tied knot was placed at the proximal end. A small incision was made on the CCA between these two sutures. A No. 4-0 suture was inserted through the incision of CCA and slowly and gently pushed into the ICA. The threading was halted temporarily when reaching the artery clamp of ICA. The pre-ligation suture was further tightened. The artery clamp blocking the blood flow in the ICA was removed, allowing the suture occlusion to be advanced into the ICA until it entered the intracranial region. When the suture was inserted about 18mm away from the bifurcation of the CCA, slight resistance was felt, which shows the head end of the suture already entered the anterior cerebral artery (ACA), and the opening of the middle cerebral artery was already blocked by the side wall of the suture occlusion. The insertion was stopped at the moment, and the time was recorded. The artery clamp on the CCA was removed and the incision was closed after no active bleeding was observed. The ischemic rats were placed at room temperature to keep their body temperature at 37°C, and anesthesia induction could be carried out 120min later. The suture was slowly and gradually withdrawn under anesthesia until the proximal end retracted into the ECA, thereby initiating reperfusion of the middle cerebral artery. Animals received the designated dosage immediately (within 10 min) after reperfusion. This dosing regimen continued once daily for a duration of 28 days, with the day of operation being defined as D1 (Day 0). A total of 5 groups were set, sham surgery group, model control group, NBP group (60mg/kg , p.o., q.d. ), compound (I) low dose p.o. group (6mg/kg, p.o., q.d.), and a compound (I) high dose p.o. group (20mg/kg, p.o., q.d.). During administration, all animals were subjected to grid walking tests (D7, D14, D21, and D28, 4 times in total) and novel object recognition tests (adaptation for novel object recognition on D26, test on D27). After the completion of the 28-day dosing period, all surviving animals in the study were euthanized, and their brains were rapidly collected for pathological analysis.

(1) The results of the grid walking tests showed that: one week after the operation, the misstep frequency of animals in the model control group is 7.24±3.59 times, and the misstep frequencies of the NBP group, the compound (I) low dose group, and the compound (I) high dose group are 7.37±3.03 times, 5.33±2.33 times, and 4.23±1.44 times, respectively, wherein the misstep frequency of animals in the compound (I) high dose group significantly reduced compared with that of animals in the model control group ($p = 0.0172$); 2-3 weeks after the operation, the misstep frequency of animals in the model control group gradually reduced due to the gradual recovery of the motor function, and the misstep frequency of the compound (I) high dose group showed no statistically significant difference from that of the model control group but still demonstrated a reduced trend. 4 weeks after the modeling, the misstep frequency of animals in the model control group reduced to the level of animals in the sham surgery group. The above results demonstrate that the Compound (I) exhibits efficacy in ameliorating walking dysfunction in animals after cerebral stroke (see Table 8 for details).

Table 8 Results of Grid Walking Test

| Group | Misstep frequency (times) | | | |
|---|---|---|---|---|
| | 1W | 2W | 3W | 4W |
| Sham surgery group (12) | 0.96± 0.66 | 0.79± 0.56 | 0.88± 0.82 | 1.79± 0.92 |
| Model control group (N = 17) | 7.24± 3.59 | 5.35± 4.40 | 4.09± 3.140 | 1.59± 0.84 |
| NBP group (N = 15) | 7.37± 3.03 | 8.10± 2.97 | 6.27± 2.890 | 2.10± 1.46 |
| Compound (I) low dose group (N = 12) | 5.33± 2.33 | 6.33± 3.53 | 4.38± 2.44 | 2.58± 1.55 |
| Compound (I) high dose group (N = 13) | 4.23±1.44* | 3.23± 2.56 | 2.50± 0.81 | 1.42± 1.11 |

Note: the data in the table are all expressed as Mean±standard deviation (Mean±SD); "N" represents the number of animals in each group for statistical analysis; "1W, 2W, 3W, and 4W" represents 1 week, 2 weeks, 3 weeks, and 4 weeks after the operation, respectively; * represents $p < 0.05$ compared with the model control group.

(2) A novel object recognition test was performed on the last day of the treatment and the novel object recognition index (NRI) of animals in each group was calculated. The NRI of animals in the model control group was 54.81 ±21.94%, which was not significantly different from the familiar object recognition index (FRI), and the NRI values of animals in the NBP group, the Compound (I) low dose group, and the Compound (I) high dose group were 70.97 ±22.57%, 70.98±22.60%, and 71.66±17.06%, respectively, wherein for the NBP group, the Compound (I)low dose group, and the Compound (I) high dose group, the NRI and FRI were significantly different ($p = 0.0074$, $p = 0.0212$, and $p = 0.0009$, respectively), and the NRI values of the Compound (I) low dose group and the Compound (I) high

dose group were comparable to animals in the sham surgery group (66±10.80%). The result has shown that Compound (I) has significant effect on animals with cognitive impairment caused by stroke (see details in Table 9).

Table 9 Results of novel object recognition tests

| Group | Sniffing time (S) | | Recognition index (%) | |
|---|---|---|---|---|
| | Novel object | Familiar object | Novel object | Familiar object |
| Sham surgery group (11) | 10.52± 4.00 | 5.70± 3.19 | 66.51±10.80*** | 33.49± 10.80 |
| Model control group (N = 14) | 3.82± 3.40 | 2.70± 2.22 | 54.81± 21.94 | 45.19± 21.94 |
| NBP group (N = 13) | 4.94± 3.60 | 1.60± 1.01 | 70.97±22.57** | 29.03± 22.57 |
| Compound (I) low dose group (N = 10) | 4.74± 3.61 | 1.54± 1.10 | 70.98±22.60* | 29.02± 22.60 |
| Compound (I) high dose group (N = 13) | 5.62± 3.60 | 2.20± 1.66 | 71.66±17.06*** | 28.34± 17.06 |

Note: the data in the table are all expressed as Mean±standard deviation (Mean±SD); "N" represents the number of animals in each group for statistical analysis; * represents $p < 0.05$ compared with the model control group, ** represents $p < 0.01$ compared with the model control group, and *** represents $p < 0.001$ compared with the model control group.

(3) Pathological examination: 1) the restoration range: samples with a restoration area > 30% are considered to be good in recovery, while samples with a restoration area ≤ 30% are considered to be poor in recovery. The proportion of animals in the model control group showing good recovery was 17.6%, the proportion of animals with restoration area > 30% in the NBP group, the Compound (I) low dose group, and the Compound (I) high dose group were 21.4%, 33.3%, and 75.0%, respectively, and the Compound (I) high dose group showed the best recovery condition and was significantly different from the model control group (Chi-square, p = 0.0080); the above results show that the Compound (I) has the effect of promoting the restoration of the infarction area (see Table 10 for details); 2) the number of perfused blood vessels in the infarction restoration area: the number of erythrocyte-containing blood vessels was compared using two criteria: ≤10 and >10 vessels. For all animals in the sham surgery group, the number of blood vessels in perfused state was greater than 10, and the proportion was 100%; in the model control group, the number of animals having ≤ 10 erythrocyte-containing blood vessels in the infarction restoration area and that of animals having > 10 erythrocyte-containing blood vessels in the infarction restoration area were 10 and 7, respectively, and the proportion of animals having > 10 erythrocyte-containing blood vessels was 41.2%; in the NBP treatment group, the number of animals having ≤ 10 erythrocyte-containing blood vessels in the infarction restoration area and that of animals having > 10 erythrocyte-containing blood vessels in the infarction restoration area were 1 and 14, respectively, and the proportion of animals having > 10 erythrocyte-containing blood vessels was 93.3%, which showed statistical significance compared with that of the model control group (Chi-square, p = 0.0028); the number of animals having ≤ 10/> 10 erythrocyte-containing blood vessels in the infarction restoration area of the Compound (I) low dose group and that of the compound (I) high dose group were 0/12 and 1/12, respectively, and the number of animals having > 10 erythrocyte-containing blood vessels was significantly greater than that of the model control group (Chi-square, p = 0.0012, p = 0.0076). In addition, for the Compound (I) low dose group and the Compound (I) high dose group, the proportion values of animal samples having > 10 erythrocyte-containing blood vessels in the restoration area were 100% and 92.3%, respectively, both being no less than 90%. The above results show that the compound (I) can significantly improve the blood vessel perfusion degree of the cerebral infarction restoration area of animals with cerebral stroke after treatment for 28 consecutive days (see Table 11 for details).

Table 10 Summary of Tissue Restoration in Each Group

| Group | Number of samples | | Proportion of samples showing good recovery (%) |
|---|---|---|---|
| | Restoration area ≤ 30% | Restoration area > 30% | |
| Sham surgery group (12) | - | 12 | 100 |
| Model control group (N = 17) | 14 | 3 | 17.6 |
| NBP group (N = 14) | 11 | 3 | 21.4 |
| Compound (I) low dose group (N = 12) | 8 | 4 | 33.3 |

(continued)

| Group | Number of samples | | Proportion of samples showing good recovery (%) |
|---|---|---|---|
| | Restoration area ≤ 30% | Restoration area > 30% | |
| Compound (I) high dose group (N = 12) | 3 | 9** | 75.0 |

Note: "N" represents the number of animals in each group for statistical analysis; ** represents $p < 0.01$ compared with the model control group as determined by Chi-square.

Table 11 Summary for abundance of perfused blood vessels in each group

| Group | No. of erythrocyte-containing blood vessels | | Proportion of erythrocyte-containing blood |
|---|---|---|---|
| | ≤10 | >10 | vessels>10 (%) |
| Sham surgery group (12) | - | 12 | 100 |
| Model control group (N = 17) | 10 | 7 | 41.2 |
| NBP group (N = 15) | 1 | 14** | 93.3 |
| Compound (I) low dose group (N = 12) | 0 | 12** | 100 |
| Compound (I) high dose group (N = 13) | 1 | 12** | 92.3 |

Note: "N" represents the number of animals in each group for statistical analysis; ** represents $p < 0.01$ compared with the model control group as determined by Chi-square.

The compounds described in Examples 2 to 10 are pharmaceutically acceptable salts of the compound from Example 1. These salts can release the free base in vivo and therefore retain the same pharmacological activities as the compound of Example 1.

**Experiment 2:** Pharmacological Effects of the Tromethamine Salt from Example 10 on Stroke

The following assays demonstrate that the tromethamine salt of Example 10 demonstrates efficacy on improving ischemic neurological deficits. The assay results also show that the tromethamine salt has good bioavailability and drug effect after oral administration.

1: Pharmacodynamic study of compound herein in rat cerebral stroke model

Middle cerebral artery occlusion (MCAO) models of SD rats were constructed using the suture occlusion method to evaluate the neuroprotective effect of the compound on neurons of rats after cerebral ischemia-reperfusion. After anesthesia induction with 3.0% isoflurane in SD rats (240-270g), the operation was performed, and the right-side neck area was dissected to reveal the right common carotid artery (CCA), external carotid artery (ECA) and internal carotid artery (ICA). The ECA was ligated. The ICA was temporarily occluded. Sutures were threaded at both the proximal end and the distal end of CCA. The distal end was securely ligated, and a loosely tied knot was placed at the proximal end. A small incision was made on the CCA between these two sutures. A No. 4-0 suture was inserted through the incision of CCA and slowly and gently pushed into the ICA. The threading was halted temporarily when reaching the artery clamp of ICA. The pre-ligation suture was further tightened. The artery clamp blocking the blood flow in the ICA was removed, allowing the suture occlusion to be advanced into the ICA until it entered the intracranial region. When the suture was inserted about 18mm away from the bifurcation of the CCA, slight resistance was felt, which shows the head end of the suture already entered the anterior cerebral artery (ACA), and the opening of the middle cerebral artery was already blocked by the side wall of the suture occlusion. The insertion was stopped at the moment, and the time was recorded. The artery clamp on the CCA was removed and the incision was closed after no active bleeding was observed. The ischemic rats were placed at room temperature to keep their body temperature at 37°C, and anesthesia induction could be carried out 120min later. The suture was slowly and gradually withdrawn when the rats were maintained under anesthesia, so that the proximal end of the suture returned to the ECA, thereby initiating reperfusion of the middle cerebral artery. Animals were administered for a single dose immediately after reperfusion (within 10min). 4 groups were established, namely a model control group, a tromethamine salt of Example 10 gavage group (20 and 40 mg/kg), and a butylphthalide oral group (60 mg/kg). A single dose was administered immediately after reperfusion (within 10 minutes), followed by one dose per day for a total of 7

consecutive doses (including the first dose). Except for the initial administration, all subsequent doses were given between 4:00 PM and 7:00 PM each day. Neurological Severity Score (NSS) behavioral assessments were conducted once before modelling and at 24 hours (1 day), 72 hours (3 days), and on the 7th day after modelling. Animals were euthanized 7 days after ischemia reperfusion, and the brains were rapidly taken, frozen, and dissected for TTC staining. The normal tissues were rose-red after staining, and the infarcted tissues were white. The infarct size (%) percentage, calculated as the weight of infarcted tissues to the weight of the whole brain, is used to evaluate the degree of cerebral ischemic injury in rats.

(1) Cerebral Infarction: as shown in Table 12, seven days after operation, animals in the sham surgery control group showed no cerebral infarction; the model control group had a cerebral infarction area of 19.951±3.432%, and the positive drug group showed a cerebral infarction area of 14.241±2.912%, which was significantly lower than that of the model control group ($P \leq 0.05$). For animals treated with the tromethamine salt of Example 10 at doses of 20 and 40 mg/kg, the cerebral infarction areas were 14.717±2.541% and 13.762±2.225% respectively, both significantly lower than that of the model control group ($P \leq 0.05$). Based on the cerebral infarction area, the inhibition rates for cerebral infarction of the tromethamine salt group from Example 10 at the dose of 40 mg/kg and the positive drug group (butylphthalide, 60 mg/kg) were calculated to be 31.023±11.15% and 28.624±14.597% respectively. The above results indicate that under the conditions of this experiment, the tromethamine salt of Example 10 significantly reduces the cerebral infarction area in ischemic stroke rats at doses of 20 mg/kg and 40 mg/kg. Specifically, the effect of the 40 mg/kg dose of the tromethamine salt from Example 10 on reducing cerebral infarction is superior to that of the positive control drug (butylphthalide) at 60 mg/kg.

Table 12 Cerebral infarct size and cerebral infarction inhibition rate of experimental animals

| Group | Whole brain weight (g) | Infarct area weight (g) | Infarct Size (%) | Cerebral infarction inhibition rate (%) |
|---|---|---|---|---|
| Sham surgery control group (= 12) | 1.354± 0.118 | 0.000± 0.000 | 0.000± 0.000 | - |
| Model control group (N = 12) | 1.138± 0.07 | 0.227± 0.043 | 19.951± 3.432 | - |
| Tromethamine salt from Example 10 20mg/kg (N=11) | 1.237± 0.082 | 0.183± 0.036 | 14.717±2.541* | 26.236± 12.737 |
| Tromethamine salt from Example 10 40mg/kg (N=13) | 1.280± 0.074 | 0.176± 0.030 | 13.762±2.225* | 31.023± 11.150 |
| Positive control group (butylphthalide) 60mg/kg (N=12) | 1.237± 0.093 | 0.175± 0.032 | 14.241±2.912* | 28.624± 14.597 |

Note: the data in the table are all expressed as Mean±standard deviation (Mean±SD); "N" represents the number of animals in each group for statistical analysis; "-" represents no data for this item; * represents $p < 0.05$ compared with animals in the model control group.

(2) Neurobehavioral scoring: As shown in Table 13, the Bederson scores of all groups were 3 points after surgery, indicating successful induction of ischemia in the animals. One day after the surgery, the NSS score for the model control group animals was 7.21±1.03. The NSS scores for the positive drug and the tromethamine salt of Example 10 treatment groups showed a trend of reduction compared to the model control group, although the differences were not statistically significant.

Three days after the surgery, the NSS score for the model control group animals was 5.92±0.70. The NSS scores for the animals in the 40 mg/kg dose group of the tromethamine salt from Example 10 were significantly lower compared to the model control group ($P \leq 0.05$). The NSS score for the positive drug group (butylphthalide, 60 mg/kg) was 5.75±0.92, which showed no significant difference compared to the model control group. During the subsequent observation period, the NSS scores of the model control animals decreased to 5 and remained at or above 5. The NSS scores of the treated groups showed a continuous decreasing trend. Seven days after modelling, the NSS score for the model control group was 5.17 ±1.56. The NSS scores for the 40 mg/kg group of the tromethamine salt from Example 10 and the positive drug group (butylphthalide, 60 mg/kg) were 3.42±1.54 and 4.04±1.20 respectively. Notably, the NSS score of the 40 mg/kg group of the tromethamine salt from Example 10 was significantly lower compared to the model control group ($P \leq 0.05$). The above results indicate that under the conditions of this experiment, the tromethamine salt of Example 10 at a dose of 40 mg/kg significantly improves neurological function damage during the acute phase of ischemic stroke in rats, and its effect is better than that of the positive drug group.

Table 13: Results of Neurobehavioral Scores in Animals

| Group | Bederson | NSS | | | | |
|---|---|---|---|---|---|---|
| | | D0 | D2 | D4 | D6 | D8 |
| Sham surgery control group (= 12) | 3.00± 0.00 | 0.00± 0.00 | 0.00± 0.00 | 0.00± 0.00 | 0.00± 0.00 | 0.00± 0.00 |
| Model control group (N = 12) | 3.00± 0.00 | 0.00± 0.00 | 7.21± 1.03 | 5.92± 0.70 | 5.13± 1.58 | 5.17± 1.56 |
| Tromethamine salt from Example 10, 20mg/kg (N=11) | 3.00± 0.00 | 0.00± 0.00 | 6.73± 1.57 | 5.68± 1.27 | 4.50± 0.63 | 4.27± 1.06 |
| Tromethamine salt from Example 10, 40mg/kg (N=13) | 3.00± 0.00 | 0.00± 0.00 | 6.04± 1.35 | 4.62±1.24* | 3.88± 1.46 | 3.42±1.54* |
| Positive control group (butylphthalide), 60mg/kg (N=12) | 3.00± 0.00 | 0.00± 0.00 | 6.71± 1.36 | 5.75± 0.92 | 4.88± 1.73 | 4.04± 1.20 |

Note: the data in the table are expressed as mean ± standard deviation (Mean ± SD); "N" represents the number of animals used for statistical analysis in each group; *$P \leq 0.05$ indicates a statistically significant difference compared to the model control group; "D0" refers to one day before surgery, while "D2", "D4", "D6", and "D8" represent 1, 3, 5, and 7 days after surgery, respectively.

(3) Summary: This study shows that the tromethamine salt of Example 10 not only reduces cerebral infarction caused by stroke but also alleviates neurological function damage. In contrast, the positive control drug only demonstrates an improvement effect on cerebral infarction. The lower dose of the tromethamine salt from Example 10 (40 mg/kg) shows superior effects in inhibiting cerebral infarction and alleviating neurological function damage compared to the higher dose of the positive control drug (60 mg/kg). This suggests that the tromethamine salt from Example 10 has better efficacy than the positive control drug.

**Experiment 3:** The Efficacy of the Tromethamine Salt from Example 10 on Amyotrophic Lateral Sclerosis (ALS)

The following assays demonstrate that the tromethamine salt of Example 10 is effective in the treatment of amyotrophic lateral sclerosis (ALS).

1. Pharmacodynamic Study of the Tromethamine Salt from Example 10 on Mice with SOD1-G93A Transgenic Amyotrophic Lateral Sclerosis (ALS)

SOD1-G93A transgenic male mice of ALS model (5 weeks old) were used to evaluate the therapeutic effects of the compound on ALS disease in animals. After one week of adaptive feeding, the mice were divided into groups A: Solvent, B: Riluzole 25 mg/kg p.o., C: Tromethamine salt from Example 10 at 40 mg/kg p.o., D: Tromethamine salt from Example 10 at 60 mg/kg p.o., based on body weight, muscle tone, and performance on the rotarod test using a random number table. The dosing frequency was continuous for two weeks, with administration from Monday to Friday each week, followed by a one-week drug holiday. Behavioral assessments were conducted once every two weeks using the rotarod test and muscle tone test, while also monitoring the animals' body weight and survival rate to evaluate the therapeutic effects of the tromethamine salt from Example 10 on ALS disease.

(1) Rotarod Test Results (Table 14): The rotarod test evaluates motor endurance and coordination by measuring the time mice remain on the rotating rod. In this study, the tromethamine salt from Example 10 at a dose of 40 mg/kg significantly improved the rotarod performance ($P<0.05$) in ALS mice during the mid-stage of disease (10-16 weeks of age). The 60 mg/kg dose group showed significant improvement in rotarod performance ($P<0.001$, $P<0.05$) during the late stage of the disease. Under the experimental conditions, riluzole exhibited a positive trend in improving rotarod performance but did not reach statistical significance. Under the conditions of this experiment, the efficacy of the tromethamine salt from Example 10 is superior to that of riluzole.

Table 14: Effect of the Tromethamine Salt from Example 10 on Rotarod performance(s) in SOD1 G93AALS Mice

| Animal Age in Weeks | Solvent | Riluzole | Tromethamine salt from Example 10 (40 mg/kg) | Tromethamine salt from Example 10 (60 mg/kg) |
|---|---|---|---|---|
| 8 | 205.0± 7.569 | 232.9± 14.20 | 237.5± 14.63 | 231.4± 12.39 |

(continued)

| Animal Age in Weeks | Solvent | Riluzole | Tromethamine salt from Example 10 (40 mg/kg) | Tromethamine salt from Example 10 (60 mg/kg) |
|---|---|---|---|---|
| 10 | 221.2± 4.947 | 254.1± 9.229 | 263.6±8.950* | 249.7± 8.397 |
| 12 | 184.2± 6.676 | 233.0± 8.894 | 259.4±10.67* | 224.6± 9.470 |
| 14 | 209.1± 5.999 | 218.9± 8.639 | 248.2±8.163* | 225.5± 8.139 |
| 16 | 117.4± 14.45 | 150.8± 16.39 | 164.0±14.20* | 179.2±5.743*** |
| 18 | 23.44± 11.81 | 45.61± 14.52 | 32.87± 9.782 | 63.55±12.04* |

Note: the data in the table are expressed as mean ± standard error of the mean (Mean ± SEM). Statistical comparisons are made versus the solvent group. * indicates *P*<0.05, ** indicates *P*<0.01, and *** indicates *P*<0.001.

(2) Muscle Tone Test (Table 15): the tromethamine salt from Example 10 at 60 mg/kg significantly improved muscle tone in SOD1 G93A ALS mice aged 18 to 20 weeks. In contrast, riluzole and the 40 mg/kg dose of the tromethamine salt from Example 10 showed a trend towards increased muscle tone but did not reach statistical significance.

Table 15: Effect of the Tromethamine Salt from Example 10 on Muscle Tone (g) in SOD1 G93AALS Mice

| Animal Age in Weeks (weeks) | Solvent Group | Riluzole Group | Tromethamine salt from Example 10 (40mg/kg) | Tromethamine salt from Example 10 (60mg/kg) |
|---|---|---|---|---|
| 8 | 133.2± 6.762 | 129.8± 6.758 | 123.3± 4.830 | 140.5± 4.598 |
| 10 | 121.8± 6.384 | 120.6± 8.532 | 115.5± 5.222 | 117.7± 5.008 |
| 12 | 112.0± 3.486 | 114.2± 3.933 | 117.1± 3.641 | 119.0± 2.873 |
| 14 | 113.0± 4.430 | 121.4± 5.091 | 115.8± 3.416 | 127.4± 4.421 |
| 16 | 107.6± 3.728 | 111.5± 4.591 | 110.1± 2.868 | 122.7± 4.427 |
| 18 | 105.8± 2.436 | 121.6± 7.208 | 114.8± 5.000 | 126.4±4.252** |
| 20 | 75.38± 2.234 | 68.71± 4.333 | 86.26± 3.279 | 92.73±2.555* |

Note: the data in the table are expressed as mean ± standard error of the mean (Mean ± SEM). Compared to the solvent group, * indicates *P*<0.05, ** indicates *P*<0.01, and *** indicates *P*<0.001.

(3) Effect on survival rate: as shown in Figure 3, compared to the control group and the positive drug group, SOD1 G93A ALS mice treated with the tromethamine salt from Example 10 (at doses of 20, 40, and 60 mg/kg) exhibited longer survival periods. Specifically, the 40 mg/kg dose group showed an extension of survival by approximately 15 days.

(4) Effect on body weight: compared to the solvent group, administration of the tromethamine salt from Example 10 at all doses and riluzole had no significant effect on the body weight of the mice.

(5) Summary

The 40 mg/kg and 60 mg/kg dose groups of the tromethamine salt from Example 10 significantly improved ALS-like motor dysfunction caused by the SOD1 G93A transgene and extended the survival period of ALS mice to some extent. Under the conditions of this experiment, the positive control drug riluzole showed some improvement in neurobehavioral function in ALS mice, but there was no significant difference compared to the solvent control group. The efficacy of riluzole was weaker than that of the tromethamine salt from Example 10.

2. Pharmacodynamic study of the tromethamine salt from Example 10 on TDP-43-A315T Transgenic ALS Mice

TDP-43-A315T transgenic mice of ALS model (5 weeks old, equal numbers of males and females) were used to evaluate the therapeutic effects of the compound on ALS disease in animals. After one week of adaptive feeding, the mice were divided into groups A: Solvent, B: Riluzole 25 mg/kg p.o., C: Tromethamine salt from Example 10 at 40 mg/kg p.o., D: Tromethamine salt from Example 10 at 60 mg/kg p.o., based on body weight, muscle tone, and performance on the rotarod test using a randomized block assignment. The dosing frequency for the mice was once daily, with continuous administration for 7 days each week. After one week of dosing, there was a one-week drug holiday. Behavioral assessments were conducted once every two weeks using the rotarod test and muscle tone test, while also monitoring the animals' body weight and survival rate to evaluate the therapeutic effects of the tromethamine salt from Example 10 on ALS disease.

(1) Muscle Tone Test (Tables 16 and 17): compared to the solvent group, the tromethamine salt from Example 10 at 60 mg/kg significantly improved muscle tone in TDP-43 G93A ALS mice aged 13 to 14 weeks. In contrast, riluzole showed a trend toward improving muscle tone but did not reach statistical significance. The 20 mg/kg and 40 mg/kg doses of the tromethamine salt from Example 10 significantly improved muscle tone in female ALS mice aged 14 to16 weeks. In contrast, riluzole did not show a significant improvement in muscle tone for female ALS mice.

Table 16: Effect of the Tromethamine Salt from Example 10 on Muscle Tone (g) in Male TDP-43 A315T ALS Mice

| Animal Age in Weeks (weeks) | Solvent Group | Riluzole Group | Tromethamine salt from Example 10 (40mg/kg) | Tromethamine salt from Example 10 (60mg/kg) |
|---|---|---|---|---|
| 6 | 158.8± 5.917 | 144.0± 10.127 | 155.4± 6.495 | 175.7± 7.244 |
| 8 | 182.8± 6.418 | 152.4± 10.35 | 170.9± 6.777 | 177.0± 5.555 |
| 10 | 160.5± 8.541 | 150.5± 14.46 | 155.6± 8.359 | 155.93± 4.345 |
| 12 | 146.4± 10.79 | 145.4± 5.551 | 123.1± 18.21 | 136.8± 16.00 |
| 13 | 86.78± 29.28 | 147.8± 1.650 | 120.8± 12.91 | 149.8±3.710** |
| 14 | 80.23± 24.17 | 129.2± 1.250 | 89.72± 19.04 | 149.8±2.045** |

Note: the data in the table are expressed as mean ± standard error of the mean (Mean ± SEM). Compared to the solvent group, * indicates *P*<0.05, ** indicates *P*<0.01, and *** indicates *P*<0.001.

Table 17: Effect of the Tromethamine Salt from Example 10 on Muscle Tone (g) in Female TDP-43 A315T ALS Mice

| Animal Age in Weeks (weeks) | Solvent Group | Riluzole Group | Tromethamine salt from Example 10 (20mg/kg) | Tromethamine salt from Example 10 (40mg/kg) |
|---|---|---|---|---|
| 6 | 155.9± 4.760 | 145.2± 5.444 | 165.4± 7.815 | 155.8± 3.677 |
| 8 | 172.8± 10.54 | 177.8± 9.836 | 190.2± 14.13 | 190.6± 10.24 |
| 10 | 149.8± 6.816 | 133.6± 12.04 | 153.7± 8.355 | 147.8± 7.822 |
| 12 | 157.4± 7.212 | 161.5± 3.666 | 159.4± 13.21 | 154.3± 6.584 |
| 13 | 143.2± 6.715 | 146.4± 6.928 | 149.8± 4.815 | 148.1± 4.851 |
| 14 | 140.8± 3.035 | 143.1± 9.165 | 150.9± 6.940 | 161.6±2.816* |
| 16 | 135.6± 3.747 | 149.0± 6.827 | 159.5±7.125* | 155.2±3.339* |

Note: the data in the table are expressed as mean ± standard error of the mean (Mean ± SEM). Compared to the solvent group, * indicates *P*<0.05, ** indicates *P*<0.01, and *** indicates *P*<0.001.

(2) Rotarod Test Results (Tables 18 and 19): the 60 mg/kg dose group of the tromethamine salt from Example 10 significantly increased the rotarod performance time in male TDP-43 ALS mice aged 11 to 13 weeks. The riluzole group showed a trend toward increasing rotarod performance time at the same age range, but this difference was not statistically significant compared to the solvent group. The 20 mg/kg dose of the tromethamine salt from Example 10 also showed a certain improvement in rotarod performance time for female mice.

Table 18: Effect of the Tromethamine Salt from Example 10 on Rotarod performance(s) in Male TDP-43 A315T ALS Mice

| Dosing Schedule (weeks) | Solvent Group | Riluzole Group | Tromethamine salt from Example 10 (40mg/kg) | Tromethamine salt from Example 10 (60mg/kg) |
|---|---|---|---|---|
| 6 | 250.7± 7.962 | 250.8± 12.71 | 272.9± 7.148 | 295.6± 3.322 |
| 8 | 272.0± 14.66 | 236.4± 16.14 | 267.5± 9.357 | 275.1± 11.91 |
| 10 | 243.5± 17.57 | 234.0± 10.64 | 275.8± 10.38 | 267.3± 10.94 |
| 11 | 173.1± 28.12 | 215.6± 31.32 | 224.3± 26.08 | 263.0±12.54** |
| 12 | 141.3± 26.60 | 202.4± 41.22 | 182.1± 40.34 | 266.3±17.99*** |

(continued)

| Dosing Schedule (weeks) | Solvent Group | Riluzole Group | Tromethamine salt from Example 10 (40mg/kg) | Tromethamine salt from Example 10 (60mg/kg) |
|---|---|---|---|---|
| 13 | 99.39± 61.47 | 135.32± 76.92 | 154.2± 30.27 | 209.0±31.68** |
| Note: the data in the table are expressed as mean ± standard error of the mean (Mean ± SEM). Compared to the solvent group, * indicates *P*<0.05, ** indicates *P*<0.01, and *** indicates *P*<0.001. | | | | |

Table 19: Effect of the Tromethamine Salt from Example 10 on Rotarod performance(s) in Female TDP-43 A315T ALS Mice

| Dosing Schedule (weeks) | Solvent Group | Riluzole Group | Tromethamine salt from Example 10 (20mg/kg) | Tromethamine salt from Example 10 (40mg/kg) |
|---|---|---|---|---|
| 2 | 269.8± 12.35 | 269.8± 5.164 | 287.0± 8.449 | 273.6± 12.14 |
| 4 | 285.0± 8.609 | 258.8± 15.65 | 288.6± 7.796 | 271.4± 10.22 |
| 6 | 267.2± 13.05 | 275.3± 9.266 | 285.4± 6.626 | 269.5± 8.270 |
| 8 | 267.8± 13.06 | 272.3± 10.56 | 266.3± 14.55 | 275.6± 9.651 |
| 10 | 244.9± 13.02 | 238.4± 16.32 | 254.2± 12.62 | 260.6± 12.34 |
| 12 | 254.6± 13.75 | 252.3± 16.24 | 266.2± 14.94 | 270.6± 9.446 |
| 14 | 252.5± 19.89 | 270.6± 12.70 | 272.3± 10.62 | 256.3± 10.00 |
| 16 | 207.9± 20.82 | 255.9± 17.39 | 267.8± 16.51 | 236.5± 15.50 |
| Note: the data in the table are expressed as mean ± standard error of the mean (Mean ± SEM). Compared to the solvent group, * indicates *P*<0.05, ** indicates *P*<0.01, and *** indicates *P*<0.001. | | | | |

(3) Body weight measurement: administration of riluzole and the tromethamine salt from Example 10 at all doses had no significant effect on the body weight of the mice.
(4) Summary: the tromethamine salt from Example 10 significantly improves motor dysfunction in TDP-43 A315T transgenic ALS mice without affecting their body weight, demonstrating superior efficacy compared to the control drug riluzole.

Experiment 4: Therapeutic Effect of the Tromethamine Salt from Example 10 on Parkinson's Disease

The following assays demonstrate that the tromethamine salt from Example 10 of this invention has an effect in alleviating cognitive dysfunction associated with Parkinson's disease.

1. Therapeutic effect of the compound on MPTP-Induced mouse Parkinson's disease model

The Parkinson's disease model was established in mice via intraperitoneal injection of 1-methyl-4-phenyl-1,2,3,6-tetrahydropyridine (MPTP) at 30 mg/kg, administered twice weekly for four consecutive weeks. The therapeutic effects of the tromethamine salt from Example 10 were evaluated based on behavioral testing, striatal dopamine (DOPA) levels, and histopathological examination results.

Fifty C57 mice were block-randomized into five groups based on their performance in the pole test before modelling: the normal control group, model control group, low-dose group of the tromethamine salt from Example 10, high-dose group of the tromethamine salt from Example 10, and the positive control group (levodopa, 20 mg/kg). Drug intervention began on the day of the first MPTP injection, with daily dosing for four consecutive weeks. The doses of the tromethamine salt from Example 10 were 5 mg/kg and 20 mg/kg, while the positive control group (levodopa) received a dose of 20 mg/kg. The pole test and grip strength test were conducted within 48 hours after the last MPTP injection.

1) Pole test results

The statistical summary of the pole test results is shown in Table 19 and Figure 4. Before modelling, there was no significant difference in pole-climbing duration among the experimental groups. At the end of the study, the climbing duration for the normal control group was 8.35±0.89 s, while the model control group showed a significantly prolonged duration of 11.51±2.39 s (*P*<0.05). The positive control group had a climbing duration of 8.76±1.57 s, showing a decreasing trend compared to the model control group. The pole-climbing durations for the low-dose and high-dose

groups of the tromethamine salt from Example 10 were 9.67±1.48 s and 8.83±1.77 s respectively. Both doses showed a reduction in climbing time compared to the model control group, with the high-dose group showing a statistically significant difference ($P$<0.05). The above results suggest that the tromethamine salt from Example 10 has a significant therapeutic effect on the MPTP-induced Parkinson's disease (PD) model.

Table 19 Pole test results (s)

| Group | Sample Size | Before Modeling | Study Endpoint |
|---|---|---|---|
| Normal Control Group | 10 | 9.49± 1.45 | 8.35± 0.89 |
| Model Control Group | 7 | 9.47± 1.48 | 11.51±2.39 |
| Tromethamine salt from Example 10, low dose | 6 | 9.40± 1.51 | 9.67± 1.48 |
| Tromethamine salt from Example 10, high dose | 8 | 9.51± 1.66 | 8.83±1.77[#] |
| Positive Control Group (Levodopa) | 6 | 9.40± 1.62 | 8.76± 1.57 |

Note: the data in the figure are expressed as mean ± SD.[&] $P$<0.05 indicates a statistically significant difference compared to the normal control group, and #$P$<0.05 indicates a statistically significant difference compared to the model control group.

2) Results of Grip Strength Test

The statistical summary of the results of grip strength test is shown in Table 20 and Figure 5. Before modeling, there was no significant difference in grip strength among the experimental groups. At the study endpoint, the grip strength of the normal control group was 260.98±14.81 g, while that of the model control group was significantly lower at 210.92 ±10.00 g ($P$<0.05). The positive control group (levodopa) showed a higher grip strength of 226.26±8.39 g compared to the model control group, indicating a positive trend. The grip strength for the low-dose and high-dose groups of the tromethamine salt from Example 10 was 219.05±12.83 g and 227.16±5.85 g respectively. Both doses showed a positive trend in grip strength compared to the model control group.

Table 20 Results of Grip Strength Test (g)

| Group | Sample Size | Before Modeling | Study Endpoint |
|---|---|---|---|
| Normal Control Group | 10 | 275.67± 9.17 | 260.98± 14.81 |
| Model Control Group | 7 | 275.92± 10.48 | 210.92±10.00[&] |
| Tromethamine salt from Example 10, low dose | 6 | 276.97± 6.40 | 219.05±12.83[&] |
| Tromethamine salt from Example 10, high dose | 8 | 278.44± 8.96 | 227.16±5.85[&] |
| Positive Control Group (Levodopa) | 6 | 272.61± 9.01 | 226.26±8.39[&] |

Note: the data in the figure are expressed as mean ± SD.[&] $P$<0.05 indicates a statistically significant difference compared to the normal control group.

3) Striatal Dopamine (DOPA) Detection Results

The summary of the striatal dopamine (DOPA) detection results is shown in Table 21 and Figure 6. The striatal dopamine (DOPA) content in the normal control group was 4931.52±1607.66 ng/g, while in the model control group, it was significantly lower at 891.92±285.94 ng/g ($P$<0.05). The positive control group had a DOPA content of 1156.72±375.72 ng/g, which showed no significant change compared to the model control group. The DOPA content in the high-dose group of the tromethamine salt from Example 10 was 2731.07±868.18 ng/g, which was significantly higher compared to the model control group ($P$<0.05). The DOPA content in the low-dose group of the tromethamine salt from Example 10 was 837.82±372.19 ng/g, showing no significant difference compared to the model control group. The above results indicate that the high-dose group (20 mg/kg) of the tromethamine salt from Example 10 has a significant protective effect on dopaminergic neurons in the MPTP-induced PD model.

Table 21: Striatal Dopamine (DOPA) Detection Results (ng/g)

| Group | Sample Size | DOPA |
|---|---|---|
| Normal Control Group | 10 | 4931.52± 1607.66 |
| Model Control Group | 7 | 891.92±285.94[&] |
| Tromethamine salt from Example 10, low dose | 6 | 837.82±372.19[&] |
| Tromethamine salt from Example 10, high dose | 8 | 2731.07±868.18[#] |
| Positive Control Group (Levodopa) | 6 | 1156.72±375.72[&] |
| Note: the data in the figure are expressed as mean ± SD.[&] *P*<0.05 indicates a statistically significant difference compared to the normal control group, and [#]*P*<0.05 indicates a statistically significant difference compared to the model control group. | | |

4) Statistical Results of Tyrosine Hydroxylase (TH) Positive Fiber Content in the Striatum

The statistical summary of the TH- positive fiber content in the striatum is shown in Table 22 and Figure 7. The tyrosine hydroxylase (TH) positive fiber content in the striatum of the normal control group was 1,133,191.93±103,519.32, while in the model control group, it was significantly lower at 470,927.76±115,639.36 (*P*<0.05). The positive control group (levodopa) had a TH-positive fiber content of 536,968.78±68,050.46, which showed no significant difference compared to the model control group. The TH-positive fiber content in the high-dose group of the tromethamine salt from Example 10 was 709,627.56±100,462.19, which was significantly higher compared to the model control group (*P*<0.05). While in the low-dose group it was 526,554.39±169,680.75, showing no significant difference compared to the model control group. Consistent with the aforementioned findings, the above results indicate that the high-dose group (20 mg/kg) of the tromethamine salt from Example 10 has a significant protective effect on dopaminergic neurons in the MPTP-induced PD model.

Table 22 Statistical Results of TH-Positive Fiber Content in the Striatum

| Group | Sample Size | TH-Positive Fiber Content |
|---|---|---|
| Normal Control Group | 10 | 1133191.93± 103519.32 |
| Model Control Group | 7 | 470927.76±115639.36[&] |
| Tromethamine salt from Example 10, low dose | 6 | 526554.39±169680.75[&] |
| Tromethamine salt from Example 10, high dose | 8 | 709627.56±100462.19[&#] |
| Positive Control Group (Levodopa) | 6 | 536968.78±68050.46[&] |
| Note: the data in the figure are expressed as mean ± SD.[&] *P*<0.05 indicates a statistically significant difference compared to the normal control group, and #*P*<0.05 indicates a statistically significant difference compared to the model control group. | | |

5) Statistical Results of TH-Positive Cell counts in the Substantia Nigra Pars Compacta

The statistical summary of the TH-positive cell counts in the substantia nigra pars compacta is shown in Table 23 and Figure 8, with individual data provided in Supplementary Table 23. The number of TH-positive cells in the normal control group was 57.47±5.81, while in the model control group, it was significantly lower at 30.57±3.29 (*P*<0.05). The positive control group had 31.50±4.14 TH-positive cells, showing no significant difference compared to the model control group. The number of TH-positive cells in the high-dose group of the tromethamine salt from Example 10 was 42.42±4.36, which was significantly higher compared to the model control group (*P*<0.05). While the low-dose group had 31.22±5.97 TH-positive cells, showing no significant difference compared to the model control group. The results once again demonstrate that the high-dose group (20 mg/kg) of the tromethamine salt from Example 10 has a significant protective effect on dopaminergic neurons in the MPTP-induced PD model.

Table 23. Statistical Results of TH-Positive Cell Counts in the Substantia Nigra Pars Compacta

| Group | Sample Size | TH-Positive Cell counts |
|---|---|---|
| Normal Control Group | 10 | 57.47± 5.81 |
| Model Control Group | 7 | 30.57±3.29[&] |
| Tromethamine salt from Example 10, low dose | 6 | 31.22±5.97[&] |

(continued)

| Group | Sample Size | TH-Positive Cell counts |
|---|---|---|
| Tromethamine salt from Example 10, high dose | 8 | 42.42±4.36[&#] |
| Positive Control Group (Levodopa) | 6 | 31.50±4.14[&] |

Note: the data in the figure are expressed as mean ± SD.[&] *P*<0.05 indicates a statistically significant difference compared to the normal control group, and #*P*<0.05 indicates a statistically significant difference compared to the model control group.

Experiment Five: Therapeutic Effect of the Tromethamine Salt from Example 10 on Spinal Cord Injury

The following assays demonstrate that the tromethamine salt from Example 10 of this invention has an effect in alleviating spinal cord injury.

1: Treatment of Spinal Cord Injury (SCI) in Rats with the Compound

In a rat spinal cord injury model, the research was done about the therapeutic effect of the tromethamine salt from Example 10 on spinal cord injury and the mechanism of action was preliminarily explored.

Male SD rats were anesthetized with isoflurane, and the dorsal thoracic region was shaved. A midline incision was made over the T10 vertebra, and the skin, subcutaneous fascia, and muscle layers were dissected layer by layer to expose the spinous process and lamina, revealing the spinal cord dura mater.

The rats were positioned beneath a JK052-type impactor, and a 10 g impact rod was dropped from a height of 5 cm onto the T10 thoracic segment to establish the SCI model. Two hours after modelling, the successfully injured rats were divided into 4 groups, with 10 rats in each group. A sham-operated group (n = 10) was included as an additional control. The test drug groups received oral gavage of the tromethamine salt from Example 10 at doses of 5 and 20 mg/kg, once daily for 28 consecutive days. The positive control group received a single intravenous injection of methylprednisolone succinate (injection grade) via the tail vein at a dose of 180 mg/kg. The sham surgery group and the model control group were given an equal volume of 0.5% CMC via oral gavage. The grouping and dosing are shown in Table 24 below.

Table 24: Grouping and Dosing Methods for the Study of Compound Treatment of Spinal Cord Injury (SCI) in Rats

| Grouping | n | Dose (mg/kg) | Dosing Volume (ml/kg) | Dosing Duration (d) | Dosing Regimen/ Frequency |
|---|---|---|---|---|---|
| Sham surgery group | 10 | - | 10 ml/kg | 28 d | ig/qd |
| Model Control Group | 10 | - | 10 ml/kg | 28 d | ig/qd |
| Low-dose Test Drug Group | 10 | 5 | 10 ml/kg | 28 d | ig/qd |
| High-dose Test Drug Group | 10 | 20 | 10 ml/kg | 28 d | ig/qd |
| Positive Control Group (Methylprednisolone Succinate for Injection) | 10 | 180 | 10 ml/kg | 1 d | iv/qd |

1) Effects on BBB Scores

Rats with spinal cord injury exhibited obvious neurological dysfunction, and BBB scores were assessed at different time points. The scoring method is as follows:

BBB Locomotor Rating Scale for Rats

| | |
|---|---|
| Scoring Criteria | 0 Point: no visible hind limb movement<br>1 point: slight movement of one or two joints, usually the hip and/or knee joints<br>2 points: extensive movement of one joint or extensive movement of one joint with slight movement of another joint<br>3 points: extensive movement of two joints.<br>4 points: slight movement of all three joints in the hind limb.<br>5 points: slight movement of two joints and extensive movement of the third joint.<br>6 points: extensive movement of two joints and slight movement of the third joint.<br>7 points: extensive movement of all three joints in the hind limb.<br>8 points: able to place the paw flat on the ground when not bearing weight.<br>9 points: occasionally bears weight on the paw flat or moves with the dorsal side of the paw bearing weight, but no consistent support with the paw flat.<br>10 points: occasional paw flat weight-bearing movement is observed; no coordination between the forelimbs and hind limbs.<br>11 points: frequent paw flat weight-bearing movement is observed, but there is no coordination between the forelimbs and hind limbs.<br>12 points: frequent paw flat weight-bearing movement is observed, with occasional coordination between the forelimbs and hind limbs.<br>13 points: common paw flat weight-bearing movement is observed, with frequent coordination between the forelimbs and hind limbs.<br>14 points: sustained paw flat weight-bearing movement and coordination between the forelimbs and hind limbs are observed; or common paw flat movement with continuous coordination between the forelimbs and hind limbs, occasionally with dorsal paw movement.<br>15 points: sustained paw flat movement and continuous coordination between the forelimbs and hind limbs, with no or occasional digging during the forward movement of the forelimbs; the initial contact position of the active paw is parallel to the body.<br>16 points: continuous paw flat movement and continuous coordination between the forelimbs and hind limbs are observed in the gait, with frequent digging during the forward movement of the forelimbs; the initial contact position of the active paw is parallel to the body, followed by rotation after weight transfer.<br>17 points: continuous paw flat movement and continuous coordination between the forelimbs and hind limbs are observed in the gait, with frequent digging during the forward movement of the forelimbs; the active paw position is parallel to the body at initial contact and remains parallel after weight transfer.<br>18 points: continuous paw flat movement and continuous coordination between the forelimbs and hind limbs are observed in the gait, with sustained digging during the forward movement of the forelimbs; the active paw position is parallel to the body at initial contact and rotates after weight transfer.<br>19 points: continuous paw flat movement and continuous coordination between the forelimbs and hind limbs are observed in the gait, with sustained digging during the forward movement of the forelimbs; the active paw position is parallel to the body at initial contact and remains parallel after weight transfer, with the tail occasionally or always hanging down.<br>20 points: continuous paw flat movement, continuous coordinated gait, sustained digging with the toes, the active paw position is parallel to the body at initial contact and remains parallel after weight transfer, the trunk is unstable, and the tail is consistently raised.<br>21 points: continuous paw flat movement, continuous coordinated gait, sustained digging with the toes, the active paw position remains parallel to the body throughout the movement, the trunk is consistently stable, and the tail is continuously raised. |

A gradual recovery in BBB scores was observed over time following SCI induction. Treatment with the tromethamine salt of Example 10 at 20 mg/kg significantly improved in BBB scores from Day 7 to Day 28 (*P*<0.05-0.01). From day 14 to day 28 after administration (methylprednisolone succinate for injection) in the positive control group, the BBB scores (*P*<0.05) significantly improved. See table 25 for the results.

Table 25: Effect of the Tromethamine Salt from Example 10 on BBB Locomotor Scores in Rats (mean±SD)

| Group | n | dose (mg/kg) | Before dosing | 3 d after dosing | 7 d after dosing | 14 d after dosing | 28 d after dosing |
|---|---|---|---|---|---|---|---|
| Sham surgery group | 10 | - | 21±0 | 21± 0 | 21± 0 | 21± 0 | 21± 0 |
| Model control | 10 | - | 0± 0### | 0± 0### | 1.4± 1.2### | 2.3±1.8### | 4.7±3.0### |
| Tromethamine salt from Example 10, low dose (5 mg/kg) | 10 | 5 | 0± 0 | 0.1± 0.3 | 1.4± 2.1 | 2.2± 3.5 | 4.8± 3.8 |
| Tromethamine salt from Example 10, high dose (20 mg/kg) | 10 | 20 | 0± 0 | 0.4± 0.7 | 3.8±2.17** | 6.7±4.9* | 9.3±4.1* |
| Positive Control Group (Methylpredniso lone Succinate for Injection) | 10 | 180 | 0± 0 | 0.1± 0.3 | 2.8± 1.9 | 5.7±4.5* | 7.9±3.4* |

Note: 1. compared with the sham surgery group, ###*P*<0.001; compared with the model control group, **P*<0.05, ***P*<0.01.

2) Effects on Pathological Changes

In the sham surgery group, the spinal cord tissue structure was dense, with intact membrane structures. The gray and white matter of the spinal cord were clearly structured with distinct boundaries. The contours of neurons and glial cells were clear, the continuity of nerve fibers was good, and the arrangement of nerve cells was neat.

Compared with the sham surgery group, the spinal cord tissue in the model control group was severely damaged, with a noticeable defect visible on one side of the spinal cord. The tissue arrangement was loose and disordered, accompanied by inflammatory cell infiltration and significant neuronal vacuolation and degeneration. No significant differences were observed between the low-dose group and the model control group. In the 20 mg/kg dose group of the tromethamine salt from Example 10, more than half (4 out of 6 cases) showed a reduction in lesions. In the positive drug group (methylprednisolone succinate for injection), some (3 out of 6 cases) showed a reduction in lesions. See Figure 9 for the results.

3) Effects on Neurofilament 200 (NF200) Expression

NF200 serves as a molecular marker of mature nerve fibers. Compared with the sham surgery group, NF200 expression slightly increased after spinal cord injury in rats (*P*<0.01). Compared with the model control group, the 20 mg/kg dose of the tromethamine salt from Example 10 significantly increased NF200 expression (*P*<0.01). In the positive control group (methylprednisolone succinate for injection), there was no significant change in NF200 expression after administration (P>0.05). The results are shown in Table 26 and Figure 10.

Table 26: Effect of the Tromethamine Salt from Example 10 on NF200 Expression (mean±SD, n=6)

| Group | dose(mg/kg) | Positive Count (number per field of view) |
|---|---|---|
| Sham surgery group | - | 4.1± 4.8 |
| Model Control Group | - | |
| Tromethamine salt from Example 10, low dose | 5 | 22.5± 7.5 |
| Tromethamine salt from Example 10, high dose | 20 | 30.3±7.4** |
| Positive Control Group (Methylprednisolone Succinate for Injection) | 180 | 22.2± 6.3 |

Note: 1. compared with the sham surgery group, ###*P*<0.01; compared with the model control group, **P*<0.05, ***P*<0.01.

4) Transcriptomic Analysis of the Injured Spinal Cord

To further verify the protective or regenerative effects of the tromethamine salt from Example 10 on injured spinal cords, tissue samples were taken 1 cm upstream and downstream of the injury site for transcriptomic analysis. GSEA analysis showed that the tromethamine salt from Example 10 significantly upregulated genes related to axonal regeneration and synapses, and significantly downregulated the expression of extracellular matrix and inflammation-related genes (see Figure 11).

## Claims

1. A pharmaceutically acceptable salt of a compound of formula (I)

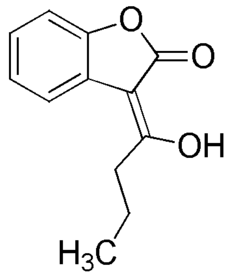

(I) ,

wherein the pharmaceutically acceptable salt is selected from sodium salt, potassium salt, magnesium salt, calcium salt, piperazine salt, ethanolamine salt, meglumine salt, or tromethamine salt, or the pharmaceutically acceptable salt is selected from monohydrate, dihydrate, trihydrate, or hemihydrate of any of the aforementioned salts.

2. The pharmaceutically acceptable salt of a compound according to claim 1, wherein the pharmaceutically acceptable salt is (Z)-3-(1-hydroxybutylidene)benzofuran-2-one sodium salt.

3. The pharmaceutically acceptable salt of a compound according to claim 1, wherein the pharmaceutically acceptable salt is (Z)-3-(1-hydroxybutylidene)benzofuran-2-one sodium monohydrate.

4. The pharmaceutically acceptable salt of a compound according to claim 1, wherein the pharmaceutically acceptable salt is (Z)-3-(1-hydroxybutylidene)benzofuran-2-one potassium salt.

5. The pharmaceutically acceptable salt of a compound according to claim 1, wherein the pharmaceutically acceptable salt is (Z)-3-(1-hydroxybutylidene)benzofuran-2-one magnesium salt.

6. The pharmaceutically acceptable salt of a compound according to claim 1, wherein the pharmaceutically acceptable salt is (Z)-3-(1-hydroxybutylidene)benzofuran-2-one calcium salt.

7. The pharmaceutically acceptable salt of a compound according to claim 1, wherein the pharmaceutically acceptable salt is (Z)-3-(1-hydroxybutylidene)benzofuran-2-one piperazine salt.

8. The pharmaceutically acceptable salt of a compound according to claim 1, wherein the pharmaceutically acceptable salt is (Z)-3-(1-hydroxybutylidene)benzofuran-2-one ethanolamine salt.

9. The pharmaceutically acceptable salt of a compound according to claim 1, wherein the pharmaceutically acceptable salt is (Z)-3-(1-hydroxybutylidene)benzofuran-2-one meglumine salt.

10. The pharmaceutically acceptable salt of a compound according to claim 1, wherein the pharmaceutically acceptable salt is (Z)-3-(1-hydroxybutylidene)benzofuran-2-one tromethamine salt.

11. A method for preparing the pharmaceutically acceptable salt of a compound of formula (I) according to any one of claims 1 to 10, comprising a step of forming a salt from a compound of formula (I) with a base.

12. The method according to claim 11, wherein a solvent used for a reaction in the salt formation reaction is selected from at least one of the following: methanol, ethanol, methyl acetate, ethyl acetate, isopropyl acetate, n-butyl acetate, acetonitrile, isopropanol, acetone, tetrahydrofuran, methyl isobutyl ketone, n-heptane, dichloromethane, toluene, isopropyl ether, methyl tert-butyl ether, n-butanol, or water.

**13.** A pharmaceutical composition comprising the pharmaceutically acceptable salt of a compound of formula (I) according to any one of claims 1 to 10 and one or more pharmaceutically acceptable carriers, diluents, or excipients.

**14.** Use of the pharmaceutically acceptable salt of a compound of formula (I) according to any one of claims 1 to 10 or use of the pharmaceutical composition according to claim 13 in preparing a medicament for treating and/or preventing ischemic brain injury-related diseases, wherein the ischemic brain injury-related diseases preferably comprise, but are not limited to cerebral ischemia, ischemic stroke, vascular dementia, post-ischemic inflammation, convulsions, ischemic neuronal injury, or necrosis.

**15.** Use of the pharmaceutically acceptable salt of a compound of formula (I) according to any one of claims 1 to 10 or use of the pharmaceutical composition according to claim 13 in preparing a medicament for treating and/or preventing neuromuscular diseases, wherein the neuromuscular diseases preferably comprise amyotrophic lateral sclerosis.

**16.** Use of the pharmaceutically acceptable salt of a compound of formula (I) according to any one of claims 1 to 10 or use of the pharmaceutical composition according to claim 13 in preparing a medicament for treating and/or preventing neurological functional impairment-related diseases, wherein the neurological functional impairment-related diseases preferably comprise, but are not limited to spinal cord injury and necrosis.

**17.** Use of the pharmaceutically acceptable salt of a compound of formula (I) according to any one of claims 1 to 10 or use of the pharmaceutical composition according to claim 13 in preparing a medicament for treating and/or preventing neurodegenerative diseases, wherein the neurodegenerative diseases preferably comprise, but are not limited to Parkinson’s disease.

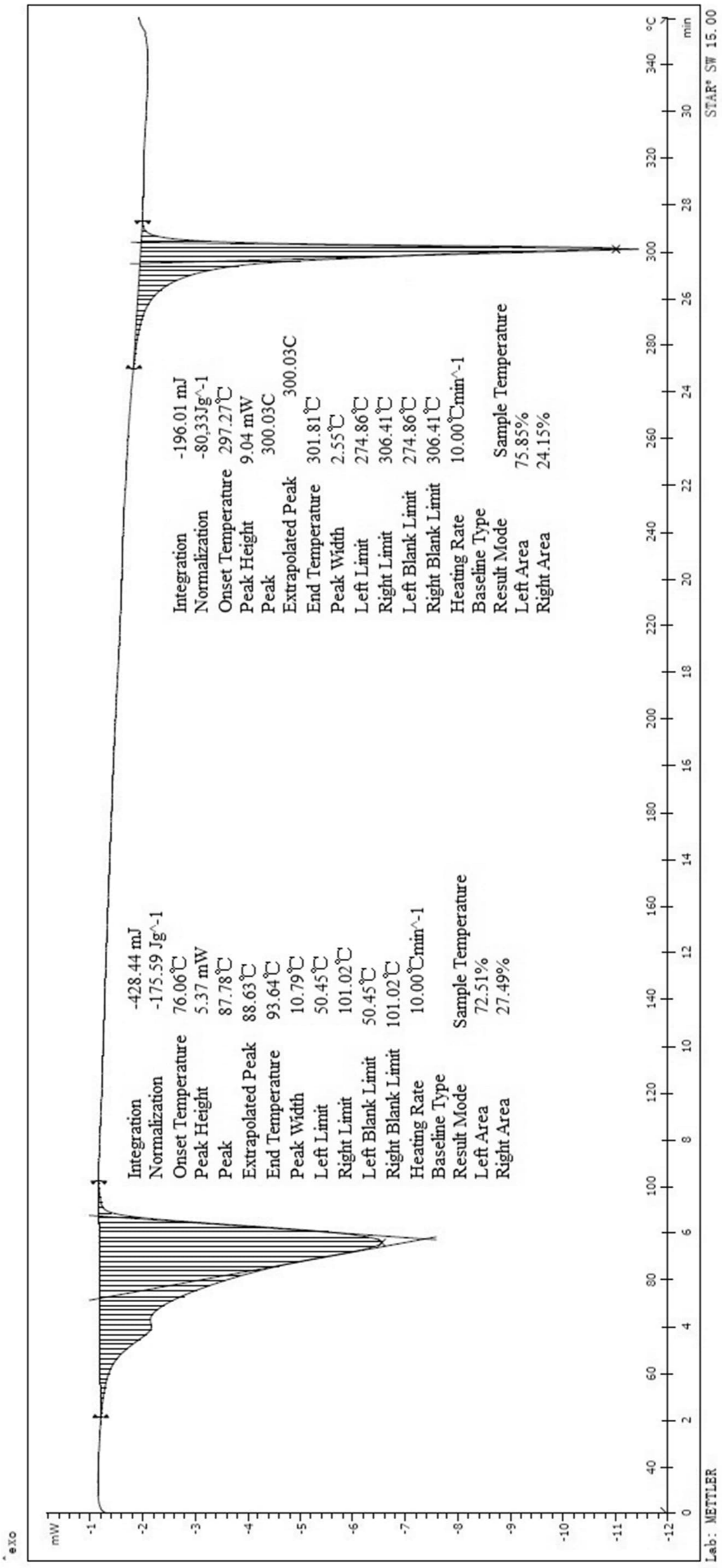
^exo
mW
Integration -428.44 mJ
Normalization -175.59 Jg^-1
Onset Temperature 76.06℃
Peak Height 5.37 mW
Peak 87.78℃
Extrapolated Peak 88.63℃
End Temperature 93.64℃
Peak Width 10.79℃
Left Limit 50.45℃
Right Limit 101.02℃
Left Blank Limit 50.45℃
Right Blank Limit 101.02℃
Heating Rate 10.00℃min^-1
Baseline Type
Result Mode Sample Temperature
Left Area 72.51%
Right Area 27.49%
Integration -196.01 mJ
Normalization -80.33Jg^-1
Onset Temperature 297.27℃
Peak Height 9.04 mW
Peak 300.03C
Extrapolated Peak 300.03C
End Temperature 301.81℃
Peak Width 2.55℃
Left Limit 274.86℃
Right Limit 306.41℃
Left Blank Limit 274.86℃
Right Blank Limit 306.41℃
Heating Rate 10.00℃min^-1
Baseline Type
Result Mode Sample Temperature
Left Area 75.85%
Right Area 24.15%
°C
min
Lab: METTLER
STARe SW 15.00

Fig. 1

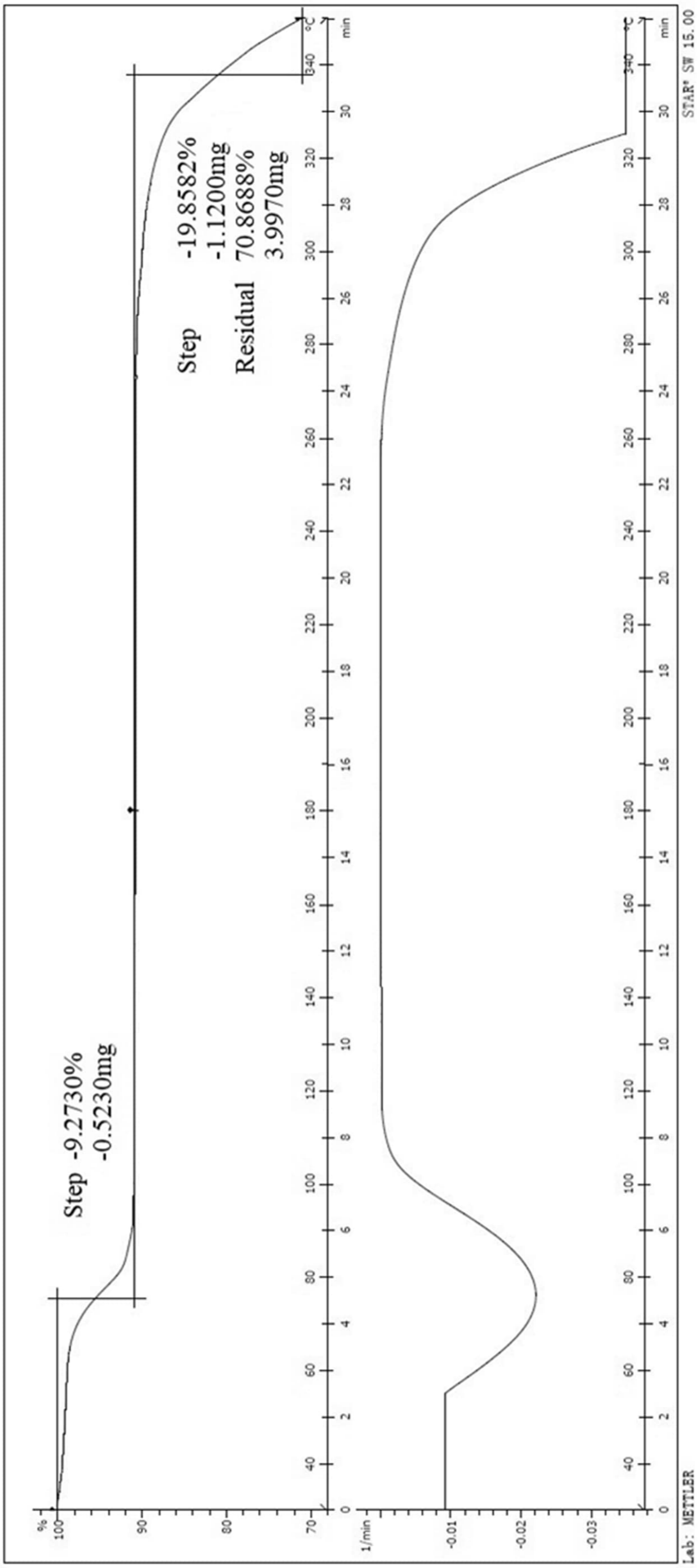
Step -9.2730%
-0.5230mg
Step -19.8582%
-1.1200mg
Residual 70.8688%
3.9970mg
Lab: METTLER
STARe SW 15.00

Fig. 2

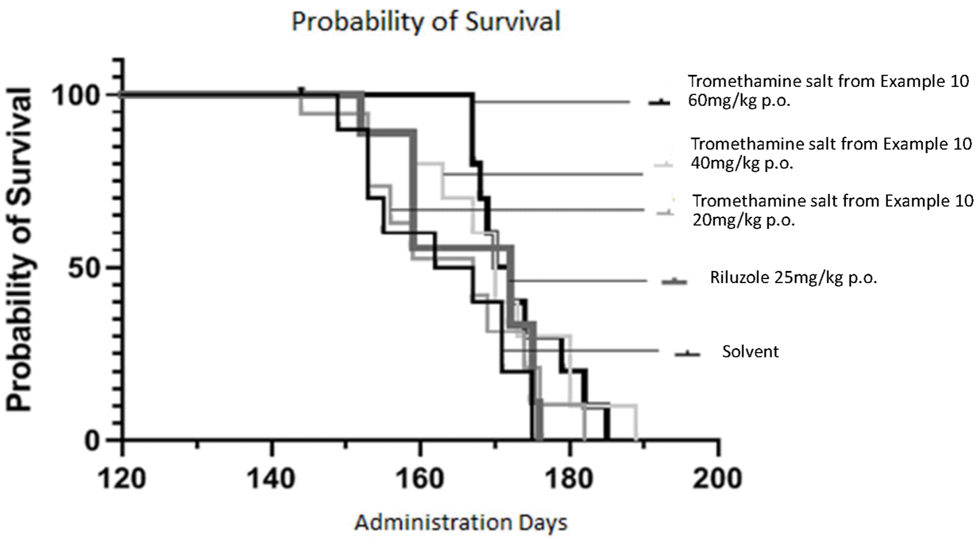
Probability of Survival
Probability of Survival
100
50
0
120
140
160
180
200
Administration Days
Tromethamine salt from Example 10 60mg/kg p.o.
Tromethamine salt from Example 10 40mg/kg p.o.
Tromethamine salt from Example 10 20mg/kg p.o.
Riluzole 25mg/kg p.o.
Solvent

Fig. 3

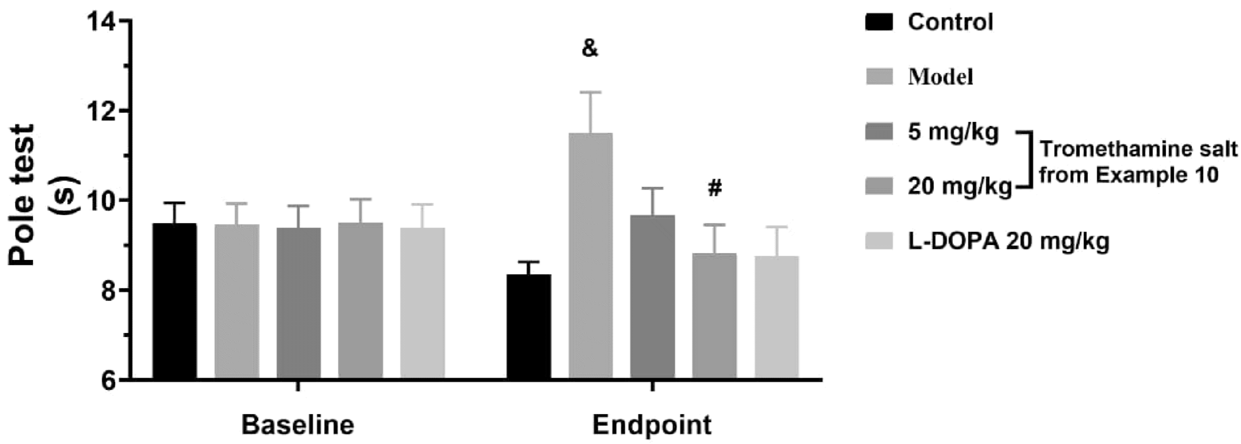
Pole test (s)
14
12
10
8
6
&
#
Baseline
Endpoint
Control
Model
5 mg/kg
20 mg/kg
Tromethamine salt from Example 10
L-DOPA 20 mg/kg

Fig. 4

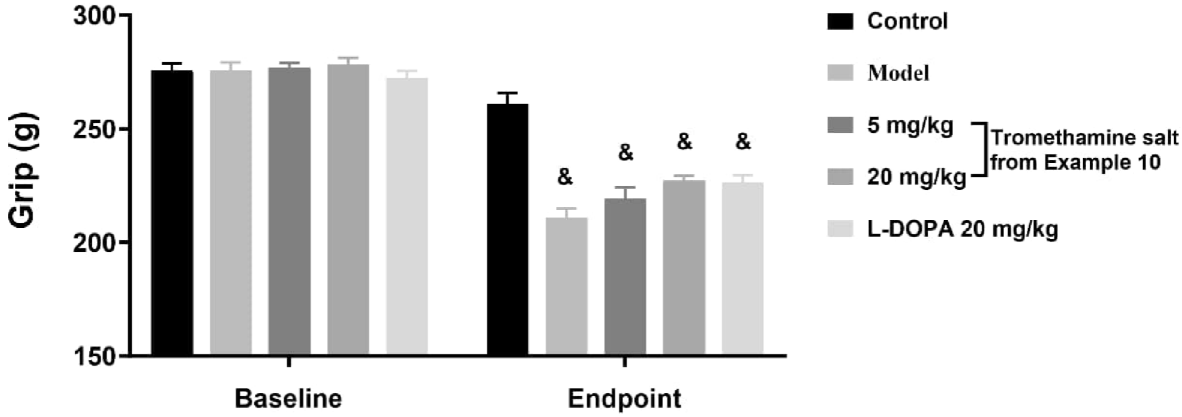
300
250
200
150
Grip (g)
&
&
&
&
Baseline
Endpoint
Control
Model
5 mg/kg
20 mg/kg
Tromethamine salt from Example 10
L-DOPA 20 mg/kg

Fig. 5

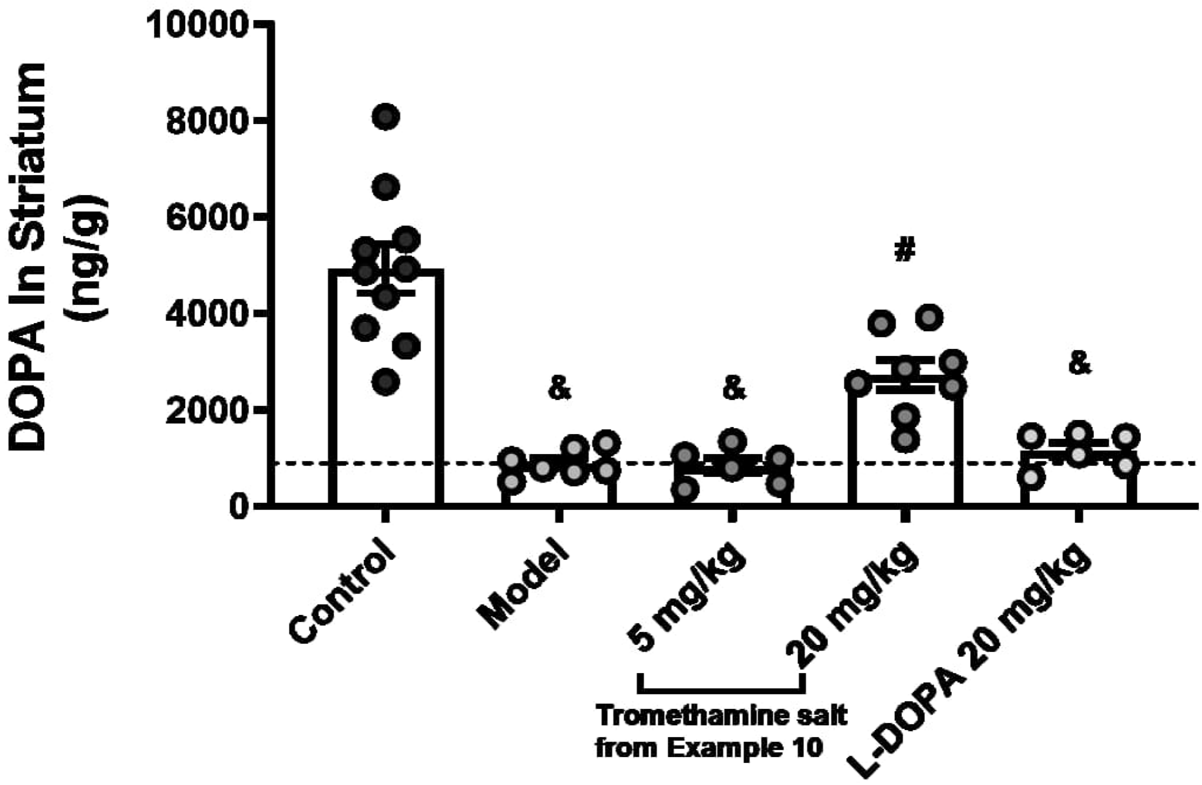
10000
8000
6000
4000
2000
0
DOPA In Striatum (ng/g)
#
&
&
&
Control
Model
5 mg/kg
20 mg/kg
L-DOPA 20 mg/kg
Tromethamine salt from Example 10

Fig. 6

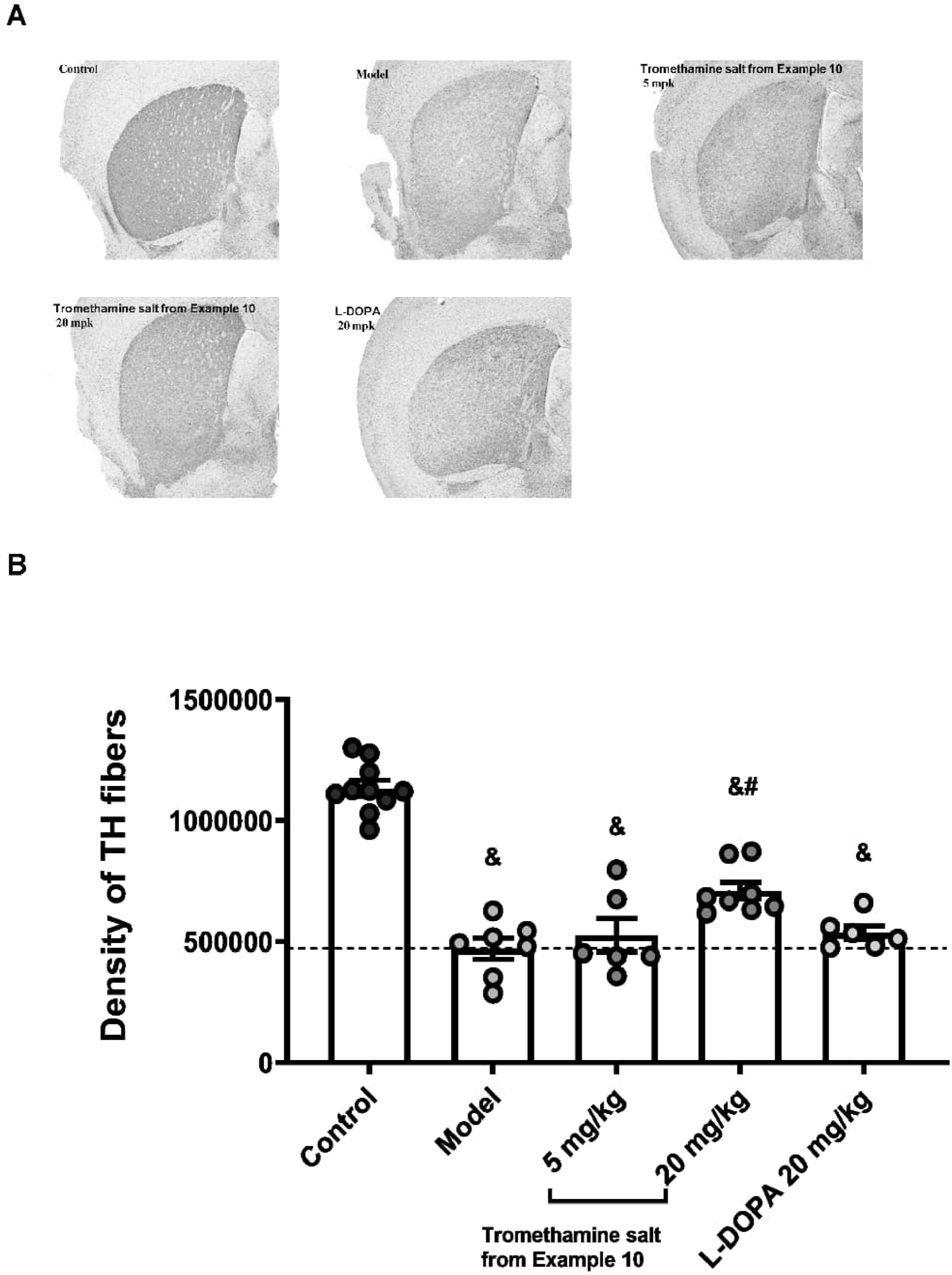
A
Control
Model
Tromethamine salt from Example 10
5 mpk
Tromethamine salt from Example 10
20 mpk
L-DOPA
20 mpk
B
Density of TH fibers
1500000
1000000
500000
0
&
&
&#
&
Control
Model
5 mg/kg
20 mg/kg
L-DOPA 20 mg/kg
Tromethamine salt
from Example 10

Fig. 7

A

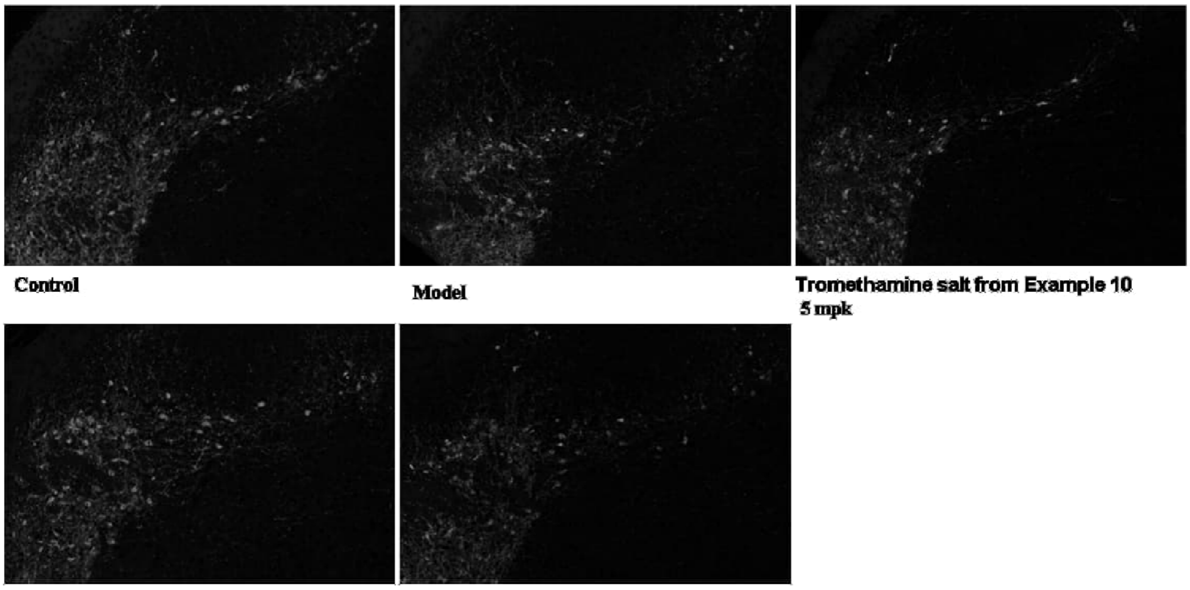
Control
Model
Tromethamine salt from Example 10
5 mpk
Tromethamine salt from Example 10
20 mpk
L-DOPA
20 mpk

B

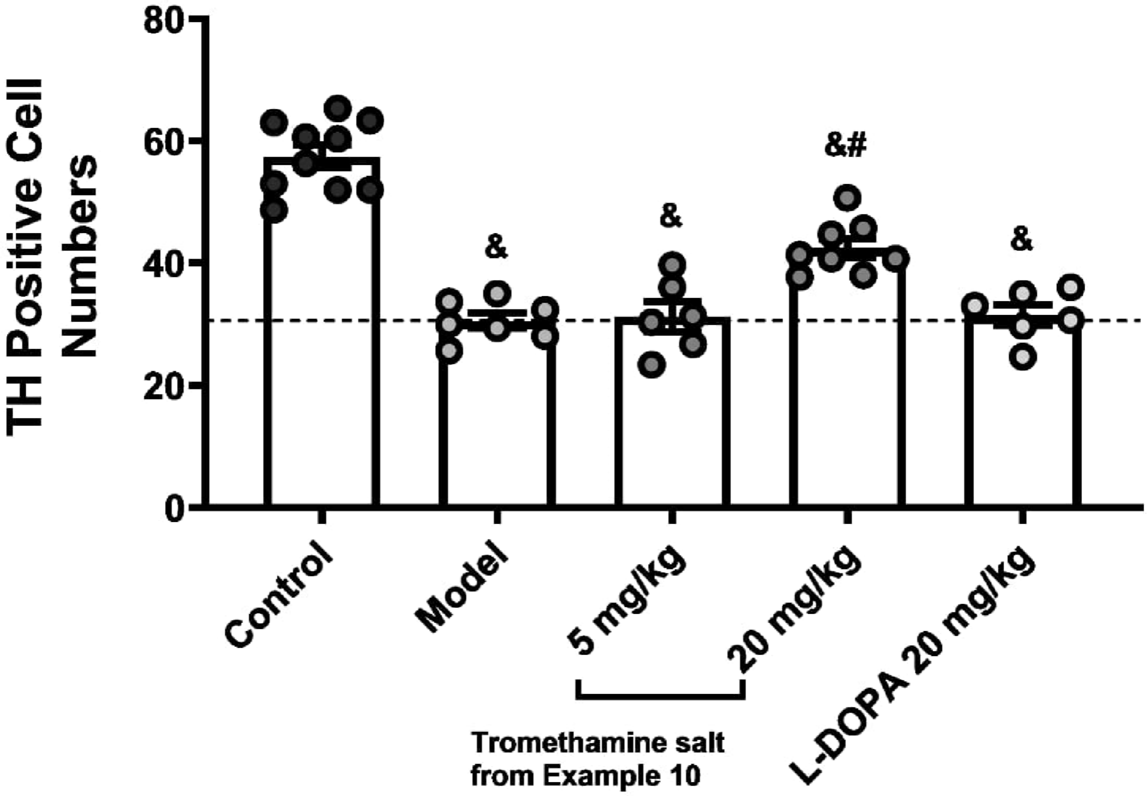
TH Positive Cell Numbers
80
60
40
20
0
&
&
&#
&
Control
Model
5 mg/kg
20 mg/kg
L-DOPA 20 mg/kg
Tromethamine salt from Example 10

Fig. 8

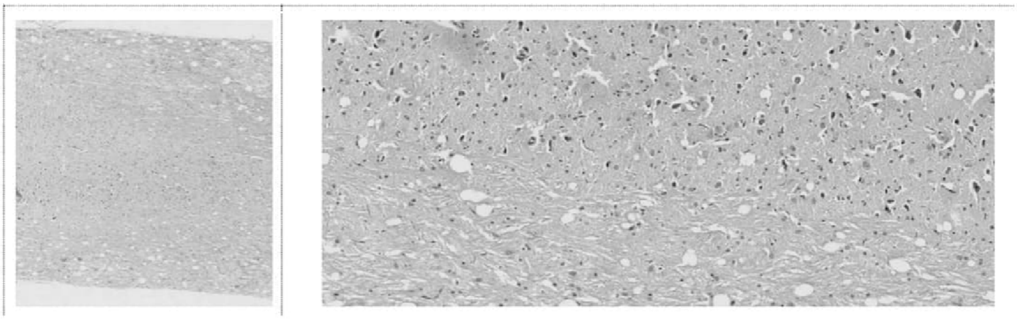

Sham surgery group

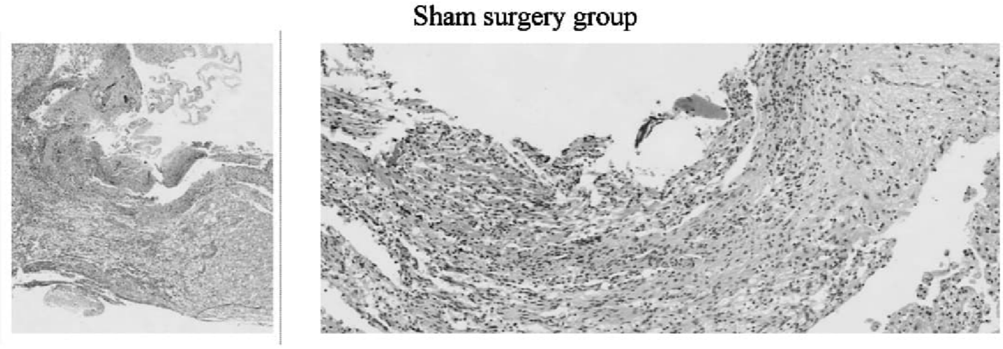

Model control group

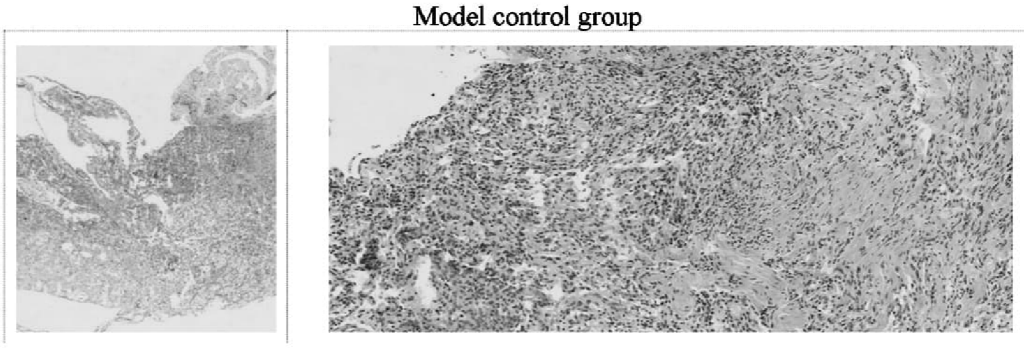

Tromethamine salt from Example 10, 5 mg/kg

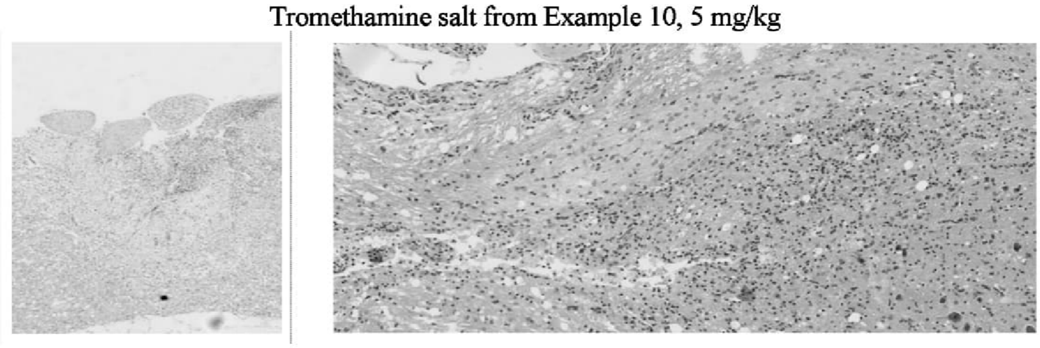

Tromethamine salt from Example 10, 20 mg/kg

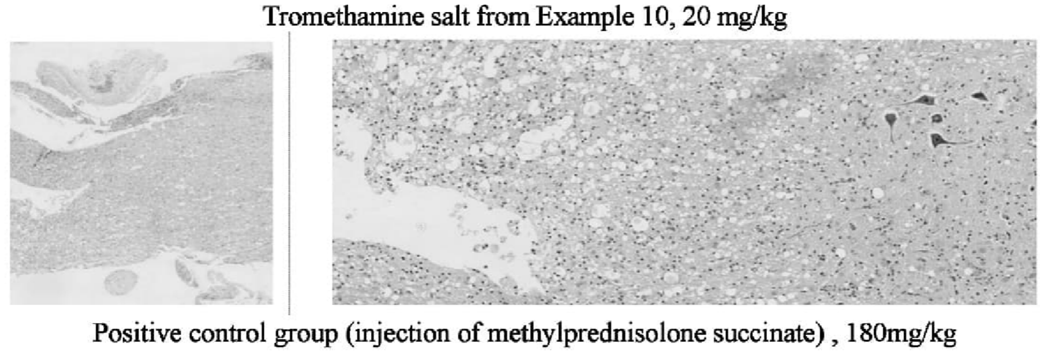

Positive control group (injection of methylprednisolone succinate) , 180mg/kg

Fig. 9

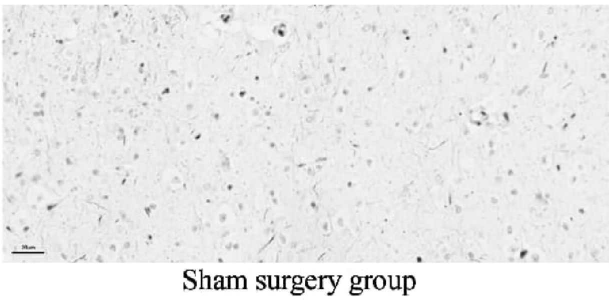

Sham surgery group

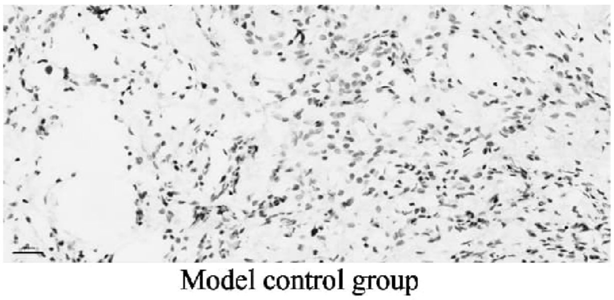

Model control group

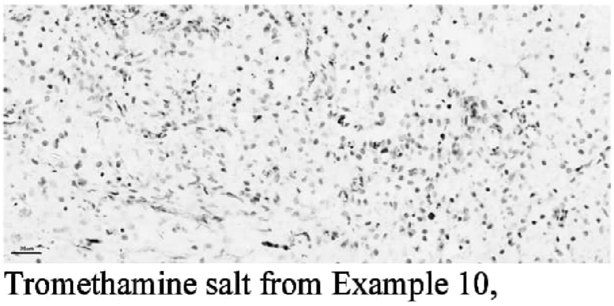

Tromethamine salt from Example 10, 5 mg/kg

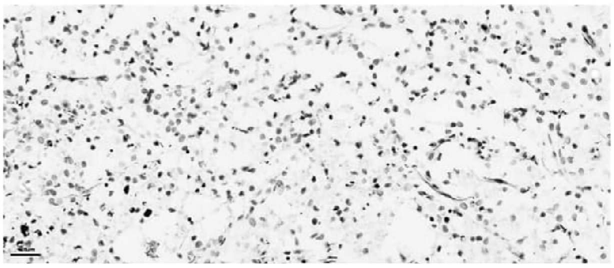

Tromethamine salt from Example 10, 20 mg/kg

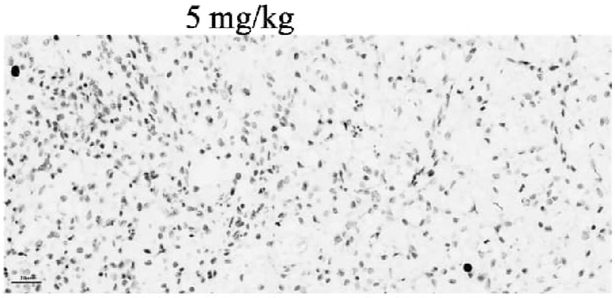

Positive control group (injection of methylprednisolone succinate) , 180mg/kg

Fig. 10

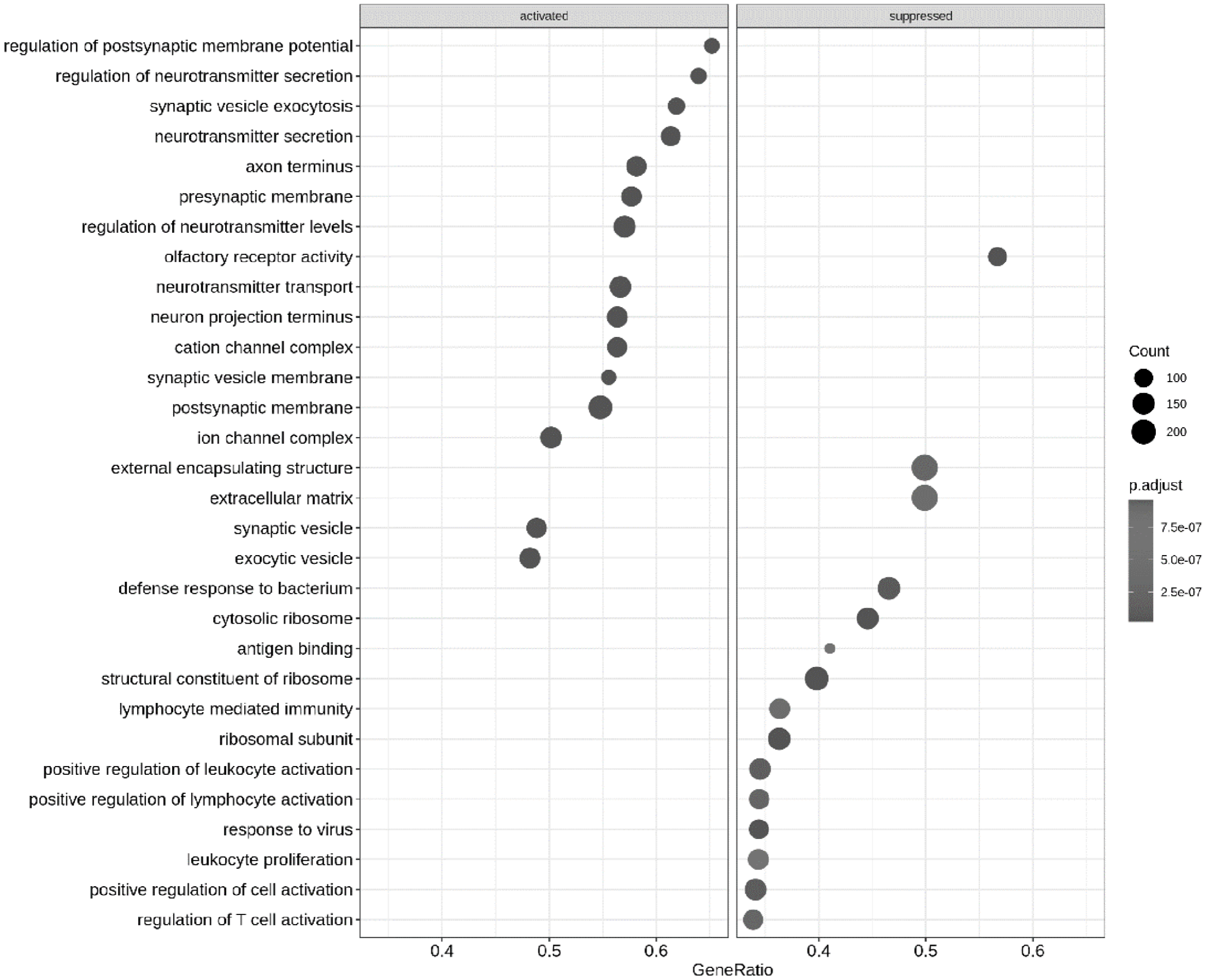
activated
suppressed
regulation of postsynaptic membrane potential
regulation of neurotransmitter secretion
synaptic vesicle exocytosis
neurotransmitter secretion
axon terminus
presynaptic membrane
regulation of neurotransmitter levels
olfactory receptor activity
neurotransmitter transport
neuron projection terminus
cation channel complex
synaptic vesicle membrane
postsynaptic membrane
ion channel complex
external encapsulating structure
extracellular matrix
synaptic vesicle
exocytic vesicle
defense response to bacterium
cytosolic ribosome
antigen binding
structural constituent of ribosome
lymphocyte mediated immunity
ribosomal subunit
positive regulation of leukocyte activation
positive regulation of lymphocyte activation
response to virus
leukocyte proliferation
positive regulation of cell activation
regulation of T cell activation
0.4
0.5
0.6
0.4
0.5
0.6
GeneRatio
Count
100
150
200
p.adjust
7.5e-07
5.0e-07
2.5e-07

Fig. 11

## INTERNATIONAL SEARCH REPORT

International application No.
**PCT/CN2024/071292**

**A. CLASSIFICATION OF SUBJECT MATTER**

C07D307/83(2006.01)i; A61K31/343(2006.01)i; A61P9/10(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS(STN), REGISTRY(STN): 江苏正大丰海制药有限公司, 朱永强, 陈琪, 唐森, 于钦策, 刘佳, 李晨辉, 苯并呋喃酮, 脑缺血, 脑卒中, 帕金森, benzofuran+one, ischemi?, parkinson

**C. DOCUMENTS CONSIDERED TO BE RELEVANT**

| Category* | Citation of document, with indication, where appropriate, of the relevant passages | Relevant to claim No. |
|---|---|---|
| PX | WO 2023001164 A1 (JIANGSU CHIA TAI FENGHAI PHARMACEUTICAL CO., LTD.) 26 January 2023 (2023-01-26)<br>claims 2-6 | 1-17 |
| A | CN 107501250 A (JIANGXI HERBFINE HI-TECH CO., LTD. et al.) 22 December 2017 (2017-12-22)<br>claims 3 and 7-10, and description, embodiment 6 | 1-17 |
| A | US 2003/0073712 A1 (GALILEO LABORATORIES, INC.) 17 April 2003 (2003-04-17)<br>claims 1, 65, and 83-84, and description, paragraphs [0157]-[0162] | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 March 2024** | **29 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/071292**

| Patent document cited in search report | | | Publication date (day/month/year) | Patent family member(s) | | | Publication date (day/month/year) |
|---|---|---|---|---|---|---|---|
| WO | 2023001164 | A1 | 26 January 2023 | AU | 2022316393 | A1 | 22 February 2024 |
| CN | 107501250 | A | 22 December 2017 | None | | | |
| US | 2003/0073712 | A1 | 17 April 2003 | US | 2006178356 | A1 | 10 August 2006 |
| | | | | AU | 2002319677 | B2 | 26 March 2009 |
| | | | | AU | 2002319677 | B8 | 30 April 2009 |
| | | | | MXPA | 04000695 | A | 26 August 2005 |
| | | | | US | 2005113416 | A1 | 26 May 2005 |
| | | | | US | 7629375 | B2 | 08 December 2009 |
| | | | | EP | 1435894 | A2 | 14 July 2004 |
| | | | | EP | 1435894 | A4 | 06 July 2005 |
| | | | | US | 2005142155 | A1 | 30 June 2005 |
| | | | | CA | 2452159 | A1 | 06 February 2003 |
| | | | | WO | 03009807 | A2 | 06 February 2003 |
| | | | | WO | 03009807 | A3 | 29 April 2004 |
| | | | | JP | 2005505519 | A | 24 February 2005 |
| | | | | JP | 4425628 | B2 | 03 March 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Chinese Journal of New Drugs and Clinical Remedies*, December 2012, vol. 31 (12), 743-747